# FASCICULE DE BREVET EUROPEEN

(11) **EP 0 807 178 B1**
(45) Date de publication et mention de la délivrance du brevet: **02.06.2004**
(21) Numéro de dépôt: 96902319.1
(22) Date de dépôt: 31.01.1996
(51) Int. Cl.: C12N 15/31, C07K 14/35, G01N 33/53, C12N 15/62, C07K 16/12, A61K 39/04

(54) **PROTEINES DE MYCOBACTERIES, MICROORGANISMES LES PRODUISANT ET LEURS UTILISATIONS VACCINALES ET POUR LA DETECTION DE LA TUBERCULOSE**
MYKROBAKTERIELLE PROTEINE, MIKROORGANISMEN WELCHE DIESE PRODUZIEREN UND IHRE VERWENDUNG ALS IMPFSTOFF UND ZUM NACHWEIS VON ZUBERKULOSE
MYCOBACTERIAL PROTEINS, MICROORGANISMS PRODUCING SAME AND USES OF SAID PROTEINS IN VACCINES AND FOR DETECTING TUBERCULOSIS

(30) Priorité: 01.02.1995 US 382184
(43) Date de publication de la demande: 19.11.1997
(73) Titulaire: INSTITUT PASTEUR, 75724 Paris Cédex 15 (FR)
(72) Inventeur: LAQUEYRERIE, Anne, F-75015 Paris (FR); MARCHAL, Gilles, F-94200 Ivry-sur-Seine (FR); PESCHER, Pascale, F-75018 Paris (FR); ROMAIN, Félix, F-91640 Fontenay-les-Briis (FR)
(74) Mandataire: Phélip, Bruno
(86) Numéro de dépôt international: PCT/FR1996/000166
(87) Numéro de publication internationale: WO 1996/023885

(56) Documents cités:
- INFECTION AND IMMUNITY, vol. 63, no. 10, Octobre 1995, WASHINGTON US, pages 4003-4010, XP002001489 LAQUEYRERIE A. ET AL.: "Cloning, sequencing, and expression of the apa gene coding for the Mycobacterium tuberculosis 45/47-kilodalton secreted antigen complex"
- INFECTION AND IMMUNITY, vol. 61, no. 2, Février 1993, WASHINGTON US, pages 742-750, XP002001490 ROMAIN F. ET AL.: "Identification of a Mycobacterium bovis BCG 45/47-kilodalton antigen complex, an immunodominant target for antibody response after immunization with living bacteria" cité dans la demande
- INFECTION AND IMMUNITY, vol. 62, no. 1, Janvier 1994, WASHINGTON US, pages 252-258, XP002001491 WIELES B. ET AL. : "Characterization of a Mycobacterium leprae antigen related to the secreted Mycobacterium tuberculosis protein MPT32" cité dans la demande
- J. EXP. MED., vol. 180, Juillet 1994, pages 319-327, XP000568830 CRISTINA M. ET AL.: "Purification, characterization, gene sequence, and significance of a bacterioferritin from Mycobacterium leprae"

## Description

La présente invention a pour objet des protéines de mycobactéries et des microorganismes les produisant.

Elle est en outre relative à l'utilisation de ces protéines et microorganismes à des fins vaccinales ou pour la détection de la tuberculose.

La tuberculose continue d'être un problème de santé publique dans le monde. Le nombre des décès annuels directement en rapport avec la tuberculose est d'environ 3 millions et le nombre des nouveaux cas de tuberculose est d'environ 15 millions. Cette mortalité due à la tuberculose est encore élevée pour les pays développés; par exemple pour la France, il est de l'ordre de 1500 par an, chiffre certainement sous-évalué d'un facteur 2 ou 3 si l'on considère les évaluations de Roujeau sur les discordances entre les chiffres officiels et les résultats d'autopsies systématiques. La récente augmentation des cas de tuberculose, ou du moins l'arrêt de la décroissance de la fréquence de cette maladie, en corrélation avec le développement de l'épidémie de VIH/SIDA, est à prendre en compte. Au total, la tuberculose reste la première maladie infectieuse par sa fréquence pour la France et les pays développés, mais surtout pour les pays en voie de développement pour lesquels elle constitue la source principale de pertes humaines en rapport avec une seule maladie.

Actuellement, le diagnostic de certitude apporté par la mise en évidence de bacilles cultivables dans un prélèvement provenant du malade n'est obtenu que pour moins de la moitié des cas de tuberculose. Même dans les cas de tuberculose pulmonaire qui représente 80 à 90% des atteints de tuberculose et qui est la forme de la maladie pour laquelle la mise en évidence de bacilles est la plus facile, l'examen des expectorations n'est positif que dans moins de la moitié des cas.

Le développement des techniques les plus sensibles, telles que la PCR (Amplification en chaîne par polymérisation), se heurte toujours à la nécessité d'obtenir un prélèvement. Les femmes et les enfants ne crachant habituellement pas, les prélèvements pour les formes de l'enfance nécessitent souvent une intervention médicale relativement spécialisée (biopsie ganglionnaire ou prélèvement par ponction lombaire du liquide céphalo-rachidien par exemple).

Par ailleurs, des inhibitions de la réaction PCR elle-même existent, de sorte qu'un prélèvement peut être inexploitable par cette technique du fait de l'impossibilité de contrôler leurs origines.

Enfin, le diagnostic bactériologique classique, examen microscopique - et culture, par sa limite de sensibilité (au mieux de l'ordre de 10⁴ à 10⁵ bacilles dans le prélèvement) suppose qu'il y ait déjà eu un développement relativement important des bacilles et donc de la maladie.

La mise en évidence d'anticorps spécifiques dirigés contre Mycobacterium tuberculosis pourrait donc apporter une aide pour le diagnostic des formes fréquentes de la maladie pour lesquelles la mise en évidence des bacilles eux-mêmes est difficile ou impossible.

Des générations successives de chercheurs ont tenté de mettre au point une technique de diagnostic sérologique de la tuberculose.

Pour une revue générale des études effectuées sur ce sujet, on se référera avantageusement à la demande PCT WO-92/21758.

Les techniques rapportées dans l'art antérieur sont majoritairement basées sur l'isolement préliminaire de protéines par leurs caractères biochimiques. Ce n'est qu'après cet isolement que les auteurs testent la capacité de ces protéines à détecter les individus atteints par la tuberculose.

La demande PCT WO 92/21758 expose une méthode pour sélectionner sans ambiguïté des antigènes représentatifs de l'infection tuberculeuse à l'aide de sérums provenant de malades atteints de tuberculose ou de cobayes immunisés par des bacilles vivants. Cette méthode qui se démarque de la majorité des expérimentations décrites dans l'art antérieur, a permis l'isolement de protéines de M.bovis présentant des poids moléculaires compris entre 44,5 et 47,5 kD.

Les dix sept acides aminés de l'extrémité N-terminale d'une de ces protéines ont été déterminés et sont les suivants:

L'article de ROMAIN et al. (1993, Infection and immunity, 61, 742-750) reprend en substance les résultats décrits dans cette demande internationale. Il décrit plus particulièrement un test ELISA en compétition utilisant un sérum immun de lapin polyclonal obtenu par immunisation de lapins à l'encontre du complexe protéique 45-47 kD mentionné ci-dessus.

Parallèlement, une banque génomique de Mycobactérium tuberculosis a été construite par JACOBS et al. (1991, Methods Enzymol, 204, 537-557).

Cette banque contient un grand nombre de clones différents.

Une protéine d'une autre espèce de mycobactéries, M. leprae a d'autre part été identifiée et séquencée par WIELES et al. (1994, Infection and Immunity, 62,252-258). Cette protéine, appelée 43 L possède un poids moléculaire déduit de la séquence nucléotidique d'environ 25,5 kDa. Son extrémité N-terminale possède une homologie de 47% avec celle du complexe protéique de 45-47 kDa identifié chez Mycobacterium bovis BCG, et dont la séquence de 17 acides aminés est reproduite ci-dessus.

Il existe, comme il a été indiqué ci-dessus, un intérêt majeur en médecine humaine, tant d'un point de vue thérapeutique que diagnostique, à identifier avec précision les protéines produites par les Mycobactéries et en particulier par M.tuberculosis.

En effet, le problème posé et non résolu jusqu'à présent réside dans l'obtention de vaccins contre un grand nombre de maladies.

Un autre problème réside dans la détection des maladies induites par les mycobactéries, telles que la tuberculose.

Le demandeur s'est donc attaché à déterminer la séquence d'une protéine de Mycobacterium tuberculosis, qui est suspectée de jouer un rôle important dans la réponse immunitaire.

Le demandeur a mis en évidence que l'ensemble des protéines correspondant au complexe 45-47 kD décrit précédemment est codé par un seul et même gène, et que la masse moléculaire calculée est différente de la masse moléculaire évaluée sur gel de polyacrylamide, du fait de la richesse en proline.

La présente invention a donc pour objet une protéine de séquence SEQ ID N° 2 ou SEQ ID N° 3 suivante:

L'invention est en outre relative à des protéines hybrides comprenant; une des séquences SEQ ID N° 2 ou SEQ ID N° 3 et une séquence d'un peptide ou d'une protéine susceptible d'induire une réponse immunitaire chez l'homme ou chez l'animal.

Avantageusement, le déterminant antigénique est tel qu'il est susceptible d'induire une réponse humorale et/ou cellulaire.

Un tel déterminant peut être de diverses natures et notamment être un fragment d'antigène protéique, avantageusement glycoprotéique, utilisé en vue d'obtenir des compositions immunogènes susceptibles d'induire la synthèse d'anticorps dirigée contre des épitopes multiples.

Ces molécules hybrides peuvent être constituées d'une molécule porteuse de séquence SEQ ID N°2 ou SEQ ID N°3 associée à une partie, en particulier un épitope de la toxine diphtérique, la toxine tétanique, l'antigène HBS du virus HBV, l'antigène VP1 du virus de la poliomyélite ou toute autre toxine ou antigène viral.

Les procédés de synthèse de molécules hybrides englobent les méthodes utilisées en génie génétique pour construire des ADN hybrides codant pour les séquences protéiques ou peptidiques recherchées.

La présente invention comprend aussi des protéines présentant des différences secondaires ou des variations limitées dans leurs séquences en acides aminés ne les modifiant pas fonctionnellement par rapport aux protéines présentant les séquences SEQ ID N° 2 et SEQ ID N°3, ou aux protéines hybrides comprenant au moins en partie ces séquences.

On notera que la présente invention a révélé une très forte différence de poids moléculaire entre le poids calculé de la protéine répondant à la séquence SEQ ID N° 3 qui est de 28779 Da et celui du complexe, évalué par gel SDS, qui est de l'ordre de 45-47 kD. Cette différence est vraisemblablement due à la haute fréquence (21,7%) de la proline dans la chaîne polypeptidique.

D'autres objets de l'invention sont les oligonucléotides, ARN ou ADN, codant pour les protéines précédemment définies. Un tel oligonucléotide présente avantageusement au moins une partie de la séquence SEQ ID N°1 suivante:

La présente invention est en outre relative à un microorganisme transformé avec un oligonucléotide tel que défini ci-dessus, ledit microorganisme produisant l'une des protéines telles que décrites ci-dessus et plus particulièrement un microorganisme sécrétant une telle protéine.

Préférentiellement, un tel microorganisme est une bactérie telle que Mycobacterium bovis BCG. Ces bactéries sont déjà utilisées chez l'homme afin d'obtenir une immunité contre la tuberculose.

La fabrication de protéines hybrides selon la présente invention dans M. bovis BCG présente des avantages particuliers. En effet, M. bovis BCG est une souche largement utilisée à des fins vaccinales et dont l'innocuité pour l'homme est reconnue. Après injection dans l'organisme humain, elle se développe lentement durant 15 jours à 1 mois, ce qui permet une excellente présentation de l'antigène contre lequel on veut obtenir une réponse à l'organisme.

On connaît par contre très mal Mycobacterium leprae, qui est l'agent de la lèpre chez l'homme. En effet, cette bactérie n'a pu être jusqu'à présent cultivée sur un milieu de culture et possède un temps de croissance très long par rapport à M bovis.

Sa pathogénicité potentielle est de plus un argument évident pour ne pas l'utiliser à des fins vaccinales.

Les protéines ayant les séquences SEQ ID N° 2 ou SEQ ID N°3 présentent l'avantage d'être reconnues par des anticorps présents chez les patients tuberculeux et constituent donc a priori des antigènes fortement immunogènes.

Les protéines sont issues de M. tuberculosis, qui est une espèce très proche de M.bovis, ces deux bactéries étant responsables de la tuberculose respectivement chez l'homme et chez les bovidés.

Les protéines issues de M.tuberculosis sont donc susceptibles de s'exprimer chez M.bovis et, pour celles possédant un peptide signal, d'être excrétées dans le milieu de culture.

M.bovis présentant les avantages indiqués ci-dessus pour la vaccination de l'homme et d'autre part les protéines répondant aux séquences SEQ ID N° 2 et SEQ ID N° 3 induisant une forte réponse immunitaire chez l'homme, il est particulièrement avantageux de produire dans M.bovis des protéines hybrides comportant une partie de la protéine issue de M.tuberculosis.

En effet, il est notoire que des antigènes de microbes pathogènes contre lesquels une vaccination est recherchée, peuvent n'induire qu'une très faible réponse chez l'homme s'ils ne sont pas présentés d'une manière spécifique.

La présente invention résoud ce problème de deux manières:
- d'une part en présentant la protéine hybride à la surface de M.bovis BCG, et/ou excrétée par la bactérie, et
- d'autre part en associant un déterminant antigénique connu pour induire une forte réponse immunitaire, c'est-à-dire le déterminant antigénique de l'une des protéines de SEQ ID N° 2 ou SEQ ID N° 3, à un déterminant antigénique induisant une faible réponse s'il est injecté seul.

L'association du déterminant antigénique d'une des protéines SEQ ID N° 2 ou SEQ ID N°3 permet d'amplifier la réponse immunitaire à l'encontre du second déterminant antigénique de la protéine hybride. Ce phénomène peut être rapproché de l'effet carrier haptène.

Il est clair qu'une telle opération n'est pas envisageable avec une protéine issue de M.leprae, telle que celle décrite dans l'article de Wieles et al. (1994, précédemment cité) car d'une part, du fait de la plus grande distance entre M. tuberculosis et M.leprae, une telle protéine risque de ne pas être convenablement exprimée et d'autre part, on connaît moins bien la réponse immunitaire induite par cette protéine de M.leprae. En outre, l'introduction d'une protéine d'une espèce pathogène à des fins vaccinales constitue un risque potentiel pour la santé humaine que les industries pharmaceutiques sont réticentes à prendre.

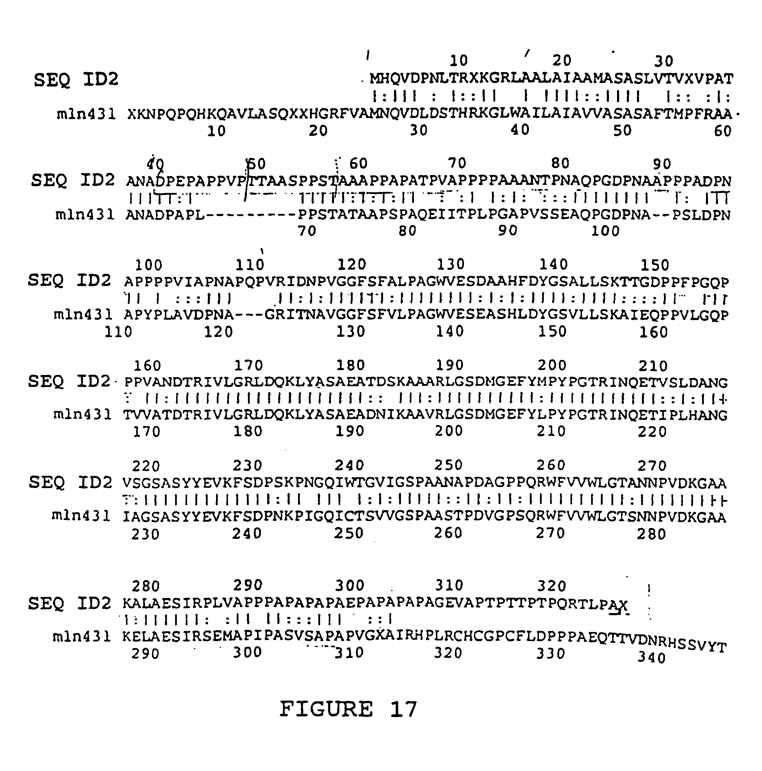

L'ensemble de ces arguments concoure donc à distinguer les protéines de séquences SEQ ID N' 2 et SEQ ID N°3 de la protéine de M.leprae décrites par Wieles et al. (1994, précédemment cité) malgré leurs homologies apparentes de séquences (voir plus loin la figure 17).

La présente invention est encore relative à des vaccins ou médicaments contenant au moins une protéine, ou un microorganisme tels que précédemment définis.

Des vaccins contenant les protéines non greffées peuvent être utilisés pour immuniser des individus à l'encontre de la tuberculose. Les protéines greffées portant un épitope provenant d'un agent biologique autre que M.bovis peuvent être utilisées pour l'immunisation contre d'autres maladies.

A titre indicatif, on pourra utiliser de 1 à 500µg de protéine par dose pour un individu, ou de 10³ à 10⁷ bactéries recombinantes par individu par voie intradermique.

La présente invention a aussi pour objet une composition pharmaceutique contenant au moins une quantité pharmaceutiquement efficace d'une protéine ou d'un microorganisme tels que précédemment définis en association avec des diluants ou des adjuvants pharmaceutiquement compatibles.

La présente invention a aussi pour objet un procédé de détection d'anticorps spécifiques de la tuberculose dans lequel un fluide biologique, dans lequel on recherche la présence desdits anticorps, est mis en contact avec une protéine telle que décrite ci-dessus.

Avantageusement, ladite protéine est fixée sur un support.

Une telle détection peut notamment être mise en oeuvre par la méthode du Western Blot (immuno-empreinte), par une méthode immunoenzymologique (ELISA) ou par une méthode radioimmunologique (RIA), à l'aide d'une trousse de dosage, contenant ces protéines ainsi que, notamment, des solutions tamponnées permettant d'effectuer la réaction immunologique et si nécessaire des substances permettant de révéler le complexe anticorps-antigène formé.

La présente invention est illustrée sans pour autant être limitée par les exemples suivants et les dessins annexés dans lesquels:
La figure 1 représente le profil de densité optique (DO) à 240 nm de la filtration moléculaire (Si 300) d'une fraction de M.tuberculosis non retenue sur une colonne échangeuse d'ions dans les conditions décrites plus loin.
La figure 2 représente le profil en densité optique à 220 nanomètres de la séparation sur colonne échangeuse d'ions (DEAE) à haute pression des molécules provenant de la fraction 1 obtenue lors de la filtration moléculaire précédente.
La figure 3 est le profil de densité optique à 220 nanomètres de la chromatographie sur colonne en phase inverse de la fraction 1 issue de la chromatographie échangeuse d'ions précédente.
Les figures 4A à 4E, sont des photographies des membranes de PVDF mises en présence respectivement:
   - d'un colorant de molécules (4A) transférées sur la membrane de PVDF. Coloration Aurodye (Amersham);
   - d'un mélange de sérums de cobayes immunisés avec des bacilles vivants (4B) ou morts (4C);
   - d'un sérum de lapin (4D) immunisé avec des antigènes purifiés à partir du BCG (Infection and Immunity (1993) 61 742-750).
   - d'un anticorps monoclonal référencé I-1081 (4E).

Ces membranes de PVDF avaient reçues préalablement les molécules des fractions séparées sur la colonne échangeuse d'ions basse pression séparées par électrophorèse en gel acrylamide. La piste O correspond au matériel brut de départ, la piste 1 à la fraction non retenue, et la piste 2 à la fraction retenue.

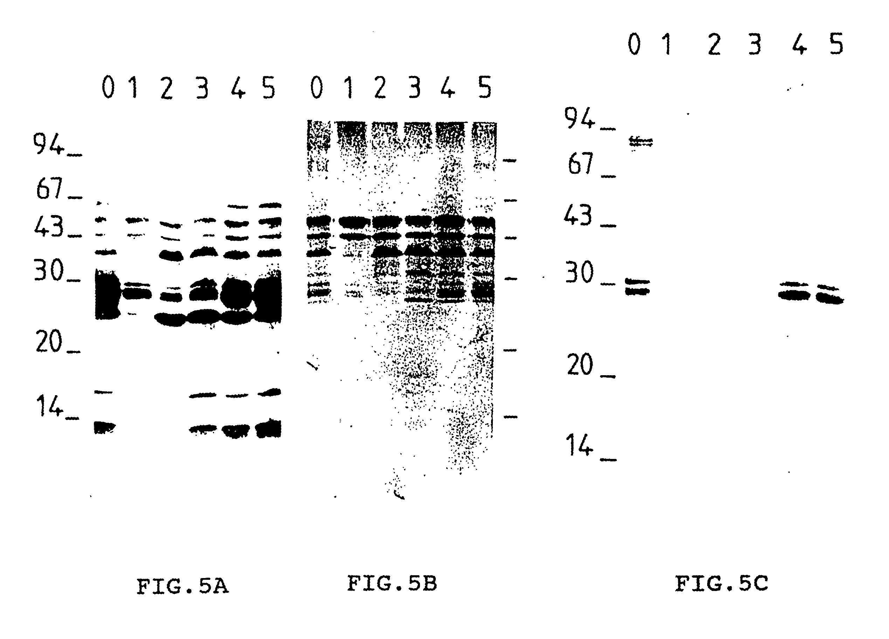

Les figures 5A à 5E représentent des membranes de PVDF correspondant à un gel obtenu par migration des 5 fractions (1 à 5) obtenues sur la colonne de gel filtration Si300 et de la fraction non retenue sur la colonne DEAE basse pression (O). Après transfert de gels identiques sur des membranes de PVDF l'une est révélée avec un colorant des protéines (Aurodye, Amersham (5A), ou avec un sérum de cobayes immunisés avec des bacilles vivants (5B), ou morts (5C), un sérum de lapin (5D) ou l'anticorps monoclonal(5E).

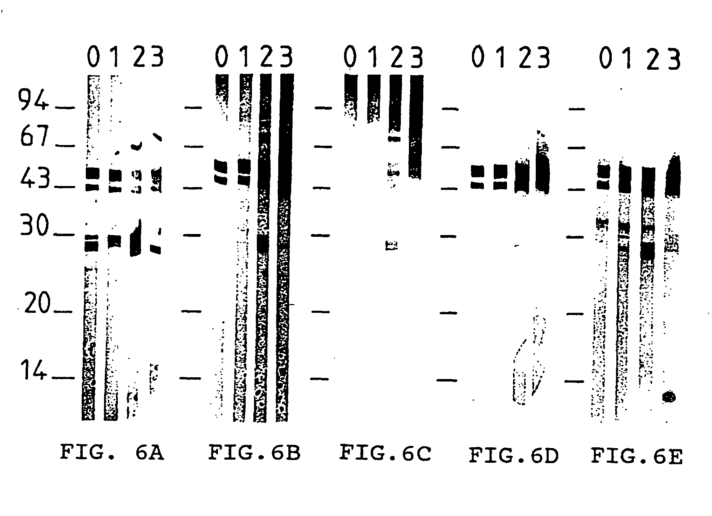

Les figures 6A à 6E représentent des membranes de PVDF correspondant à un gel obtenu par migration des fractions obtenues sur colonne échangeuse d'ions haute pression ( 1 à 3) et de la fraction 1 obtenue par filtration sur tamis moléculaire (puits 0), ladite membrane étant mise en présence:
- d'un colorant des protéines (6A),
- d'anticorps de sérums de cobayes immunisés respectivement avec des bacilles vivants (6B) ou morts (6C),
- d'un sérum de lapin (6D),
- de l'anticorps monoclonal (6E).

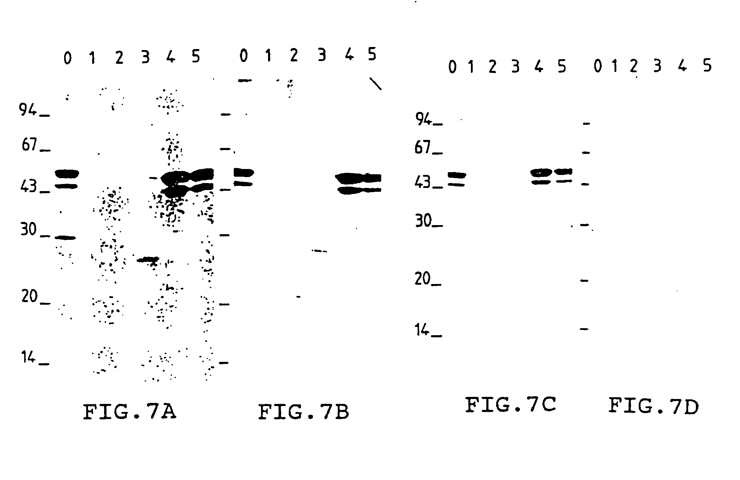

Les figures 7A à 7D représentent l'empreinte des gels sur des membranes correspondant à la migration de la fraction 1 obtenue sur colonne échangeuse d'ions (0) et des fractions obtenues par chromatographie en phase inverse (1 à 5), mises en présence des mêmes réactifs que dans les figures 6A, à 6B, 6D et 6E avec les mêmes codes.

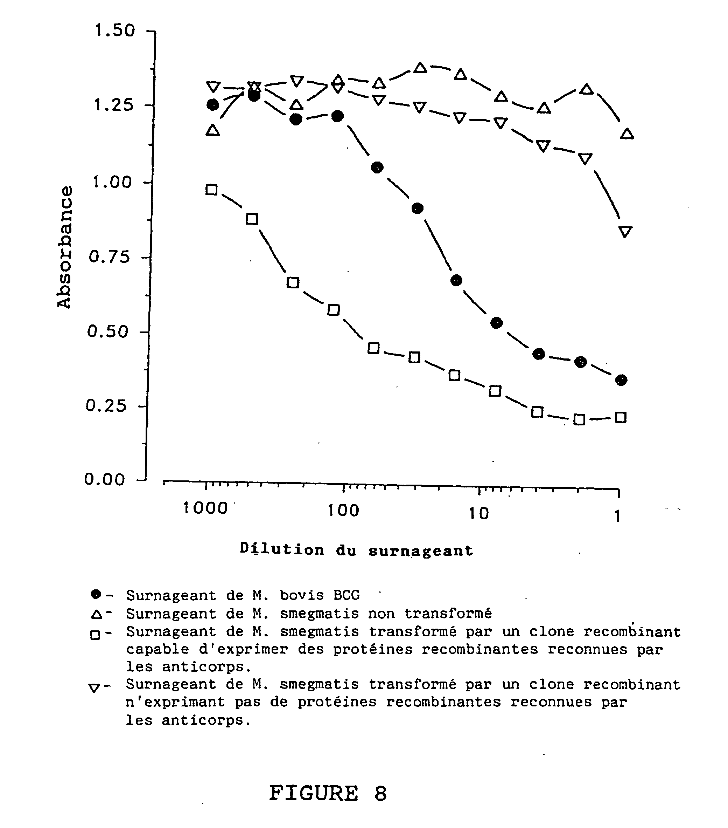

La figure 8 illustre le criblage de la banque génomique d'expression de M.tuberculosis H37Rv dans M. smegmatis. Les surnageants de M. bovis BCG, M. smegmatis non transformés et M. smegmatis transformés par des clones recombinants exprimant ou n'exprimant pas des protéines recombinantes reconnues par des anticorps ont été testés à différentes dilutions.

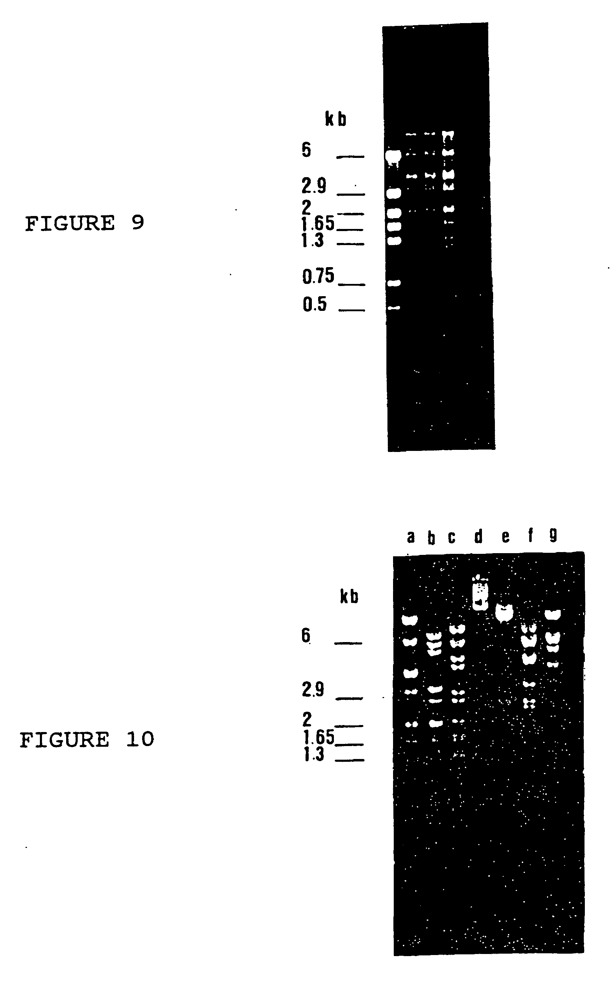

La figure 9 illustre la migration en gel d'agarose de trois cosmides sélectionnés dans la banque, électroporés dans E. coli et extraits par lyse alcaline.

La figure 10 représente la migration sur un gel de l'ADN cosmidique de pLA1 extrait de E.coli NM554 digéré par BamHI (a), SmaI (b), HpaI (c), NotI (d), SspI (e), EcoRI (f) et Hind III (g).

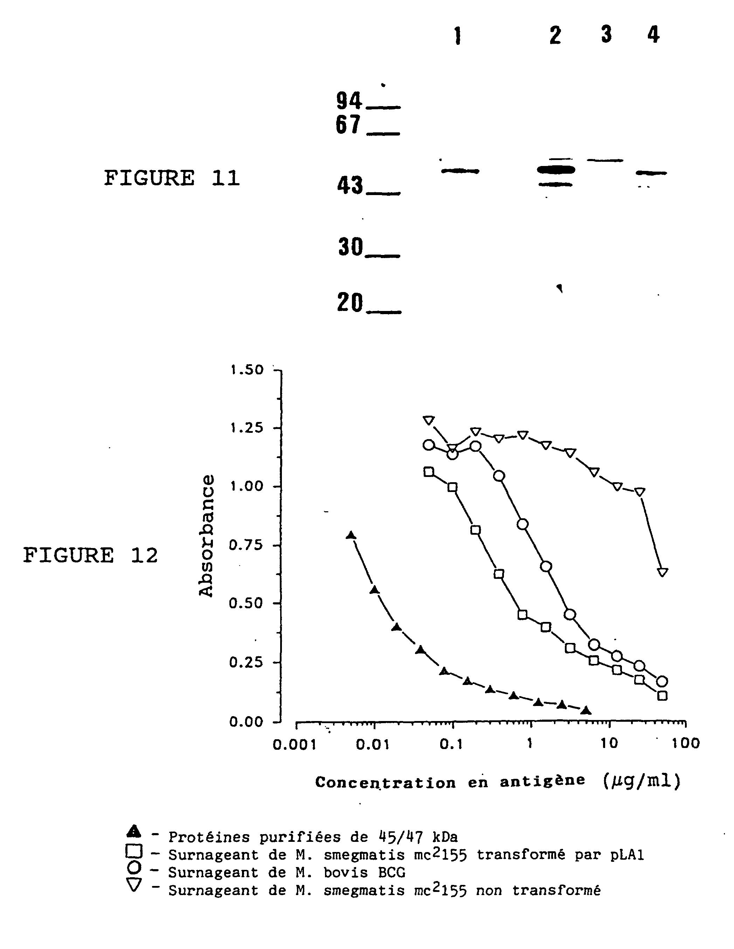

La figure 11 illustre l'expression des protéines de 45/47 kDa dans les mycobactéries. Les surnageants de culture bactérienne de 7 jours ont été lavés et concentrés sur une membrane Amicon PM10, lyophilisés et analysés en immuno-empreinte. Les protéines sont révélées par des anticorps polyclonaux d'un sérum de lapin dilué au 1/500è.

Sont déposés par puits:
(1) 0,25 µg de protéines de 45/47 kDa purifiées de M.bovis BCG,
(2) 5 µg de surnageant de M. smegmatis mc²155 transformé par pLA1,
(3) 5 µg de surnageant de M. smegmatis mc²155 non transformé,
(4) 5 µg de surnageant de M.bovis BCG.

La figure 12 illustre l'expression des protéines de 45/47 kDa dans les mycobactéries. Les surnageants de culture bactérienne ont été lavés et concentrés sur une membrane Amicon PM10, puis lyophilisés et analysés dans un test Elisa en compétition. Différentes concentrations des surnageants lyophilisés sont mises en présence d'une dilution au 1/8000è du sérum polyclonal de lapin, puis ce mélange est transféré dans des puits sur lesquels sont fixées les protéines purifiées.

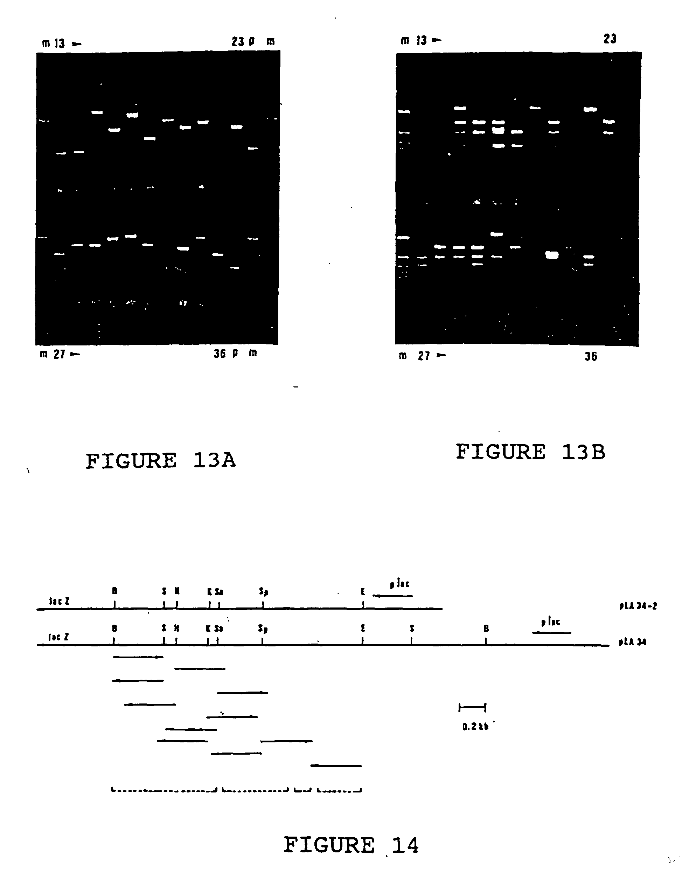

Les figures 13A et 13B sont des profils plasmidiques (13A) et profils de restriction BamH I (13 B) de différents clones recombinants pUC18::M.tuberculosis H37Rv, obtenus par ligation des fragments d'une digestion BamH I du cosmide pLA1 dans pUC18. Sur cette figure sont présentés 21 des 36 clones étudiés. Les puits "p" correspondent au vecteur témoin pUC18, les puits "m" correspondent aux marqueurs de taille qui sont des fragments du plasmide pKN coupés par Pvu II.

La figure 14 est la carte de restriction des inserts permettant l'expression des protéines de 45/47 kDa dans E.coli. Un ensemble de clones a été obtenu par des délétions à partir des plasmides pLA34 et pLA4, contenant l'insert de 3 kb cloné dans les deux orientations. Les flèches indiquent la direction de la détermination des séquences à partir de ces clones à l'aide des amorces "directes" et "inverses". B, BamH I S, Sma I E, EcoR I K Kpn I H, Hind III Sa, Sal I Sp, Sph I

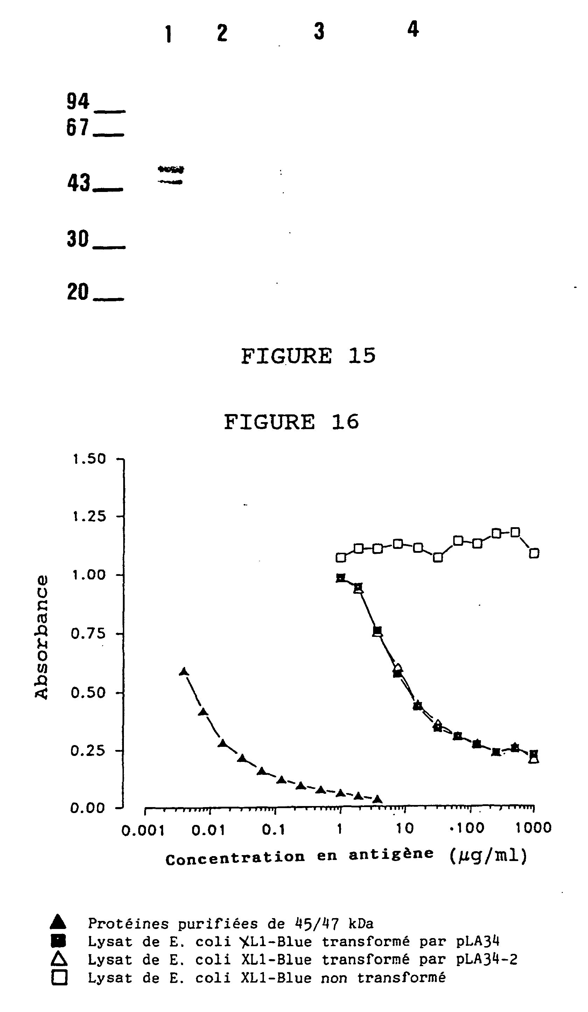

La figure 15 illustre l'expression des protéines de 45/47 kDa dans E.coli. Les lysats de culture bactérienne ont été analysés en immuno-empreinte.

Les protéines sont révélées par des anticorps polyclonaux de lapin purifiés sur DEAE, puis absorbées sur un lysat de E. coli immobilisé sur colonne Sepharose-4B activé par du bromure de cyanogène.

Sont déposés par puits:
(1) 0,2 µg de protéines purifiées de 45/47 kDa,
(2) 25 µg de lysat de E.coli XL-Blue transformé par pLA34-2,
(3) 25 µg de lysat de E.coli XL-Blue transformé par pLA34,
(4) 25 µg de lysat de E.coli XL1-Blue non transformé.

La figure 16 illustre l'expression des protéines de 45/47 kDa dans E.coli. Les lysats de culture bactérienne, analysés par un test Elisa en compétition ont été utilisés sous forme brute.

La figure 17 est une comparaison de séquence SEQ ID N°2 selon l'invention et de la séquence de la protéine de M.leprae (mln 431).

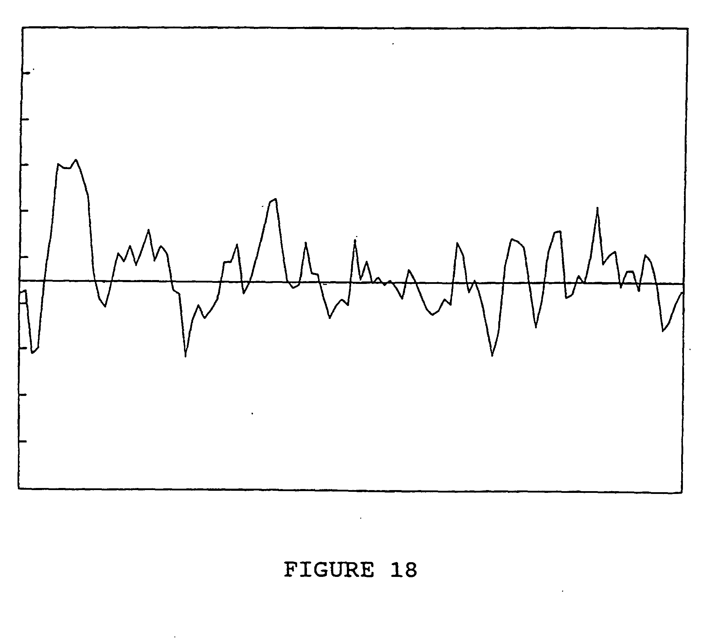

La figure 18 est un profil d'hydrophobicité de la protéine de séquence SEQ ID N°2.

### EXEMPLE 1:

### Procédé de purification des antigènes de M.tuberculosis

### 1) Obtention des antigènes:

Des cultures de M.tuberculosis (souche H37Rv) sont effectuées en ballons contenant 130 ml de milieu synthétique de Sauton selon une technique classique décrite pour la culture du BCG (Gheorghiu et al. Bull. Institut Pasteur 1983, 81: 281-288). Le milieu de culture est récolté après 20 jours à 37°C, décanté et filtré (0,22 µm) à la température du laboratoire. Ces opérations sont réalisées dans une boîte à gants pour des raisons de sécurité. Le milieu de culture récolté et filtré est de nouveau filtré sur filtre 0,22 µm sous hotte de sécurité avant d'être utilisé pour les opérations suivantes:

Placé sur une membrane Amicon (PM10) sous une pression d'azote de 2 bars et à 4°C, le milieu de culture est lavé intensivement avec de l'eau rétro-osmosée contenant 4% de butanol, puis concentré 10 à 20 fois par rapport au volume initial. Ce milieu de culture concentré, contenant les molécules non exclues par la membrane PM10 Amicon, est lyophilisé, pesé et conservé sous forme de poudre à -20°C. Les 12g du matériel de départ -utilisés pour le schéma de purification décrit ci-dessous sont obtenus à partir de 70 litres de milieu de culture.

### Schéma de purification:

### 2) Colonne échangeuse d'ions à basse pression:

Une colonne échangeuse d'ions préparative à basse pression de hauteur 300 mm et de diamètre 32 mm est préparée avec environ 240 ml de gel Trisacyl M (SEPRACOR). Elle est équilibrée avec une solution saline tamponnée (Na₂ HPO₄/NaH₂PO₄ 10 mM, pH = 7, et NaCl 10 mM) contenant 4% de butanol.

Le matériel concentré et lyophilisé préparé à l'étape antérieure est solubilisé (dans la solution saline tamponnée précédente) puis ultracentrifugé - durant 120 mn à 40.000 G. Seule la partie supérieure (4/5) de la solution centrifugée est reprise et appliquée sous le contrôle de la pompe péristaltique sur la colonne échangeuse d'ions. Une première fraction majoritaire non retenue sur la colonne est recueillie. Une seconde fraction est obtenue après élution de la colonne avec une solution saline tamponnée (Na₂HPO₄) NaH₂ et 10 mM, pH = 7,5 et NaCl 1M). Chaque fraction, placée sur une membrane Amicon (PM10) sous une pression de 2 bars et lavée intensivement avec de l'eau rétro-osmosée contenant 4% de butanol, est concentrée environ 15 fois. La fraction non retenue par la colonne contient 2,9 g de matériel et l'essentiel des molécules qui sont purifiées dans les étapes suivantes. La fraction retenue par la colonne puis éluée par la solution concentrée en sel contient environ 1,01 g de matériel.

### 3) Filtration sur gel

Une colonne préparative haute pression Si 300, 3 µm de 50 x 750 mm (SERVA), est équilibrée par passage d'une solution saline tamponnée (Na₂HPO₄ 50 mM ajustée à pH 7,5 avec-KH₂PO₄) contenant 4% de butanol; cette solution est préalablement filtrée sur une membrane (0,22 µm). Le débit de la colonne est ajusté à 1,25 ml bar par mn; la pression maximum réglée à 45 bars n'est pas atteinte.

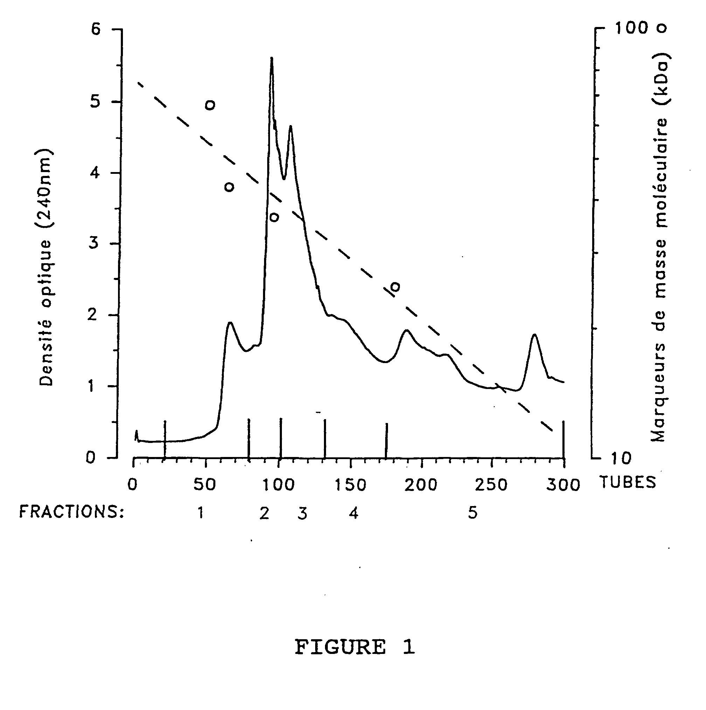

Le matériel à injecter sur la colonne est préparé à la concentration de 50 mg/ml dans la solution tampon/butanol. Des échantillons de 10 ml sont préparés et congelés à -20°C. Chaque échantillon de 10 ml, filtré de nouveau après décongélation et injecté sur la colonne, contient environ 500 mg de matériel brut. Les profils des densités optiques à 240 nm sont rapportés figure 1 pour une séquence typique de séparation. Les cinq fractions principales choisies d'après le profil sont concentrées à 4°C et lavées intensivement sur membrane Amicon PM10 avec de l'eau rétro-osmosée contenant 4% de butanol. Chaque fraction concentrée est lyophilisée, pesée puis conservée à - 20°C. La fraction 1 de cette étape contient les molécules principales reconnues par les anticorps des cobayes immunisés avec des bacilles vivants ou par les anticorps des malades tuberculeux. Seule cette fraction est soumise à l'étape suivante.

### 4) Colonne échangeuse d'ions:

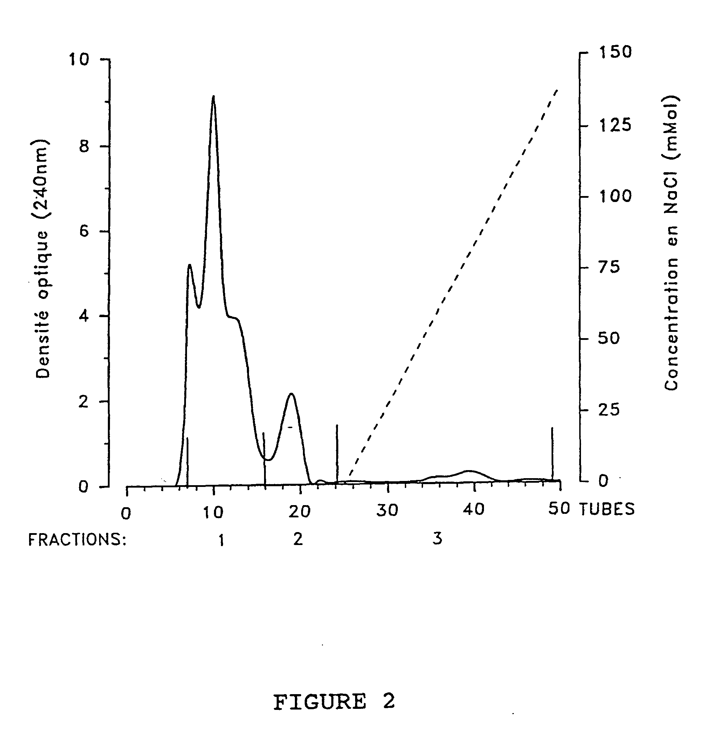

Une colonne préparative DEAE-TSK 5PW de 21,5 x 150 mm (LKB) est équilibrée avec une solution saline tamponnée (Na₂HPO₄/NaH₂PO₄ 10 mM, pH = 7,5 et NaCl 10 mM) contenant 4% de butanol. La pression maximum est inférieure à 30 bars pour un débit de 6 ml/mn. Pour le tampon d'élution, seule la concentration de NaCl est modifiée (1 M). Un gradient linéaire est appliqué selon le schéma indiqué figure 2 après injection d'un volume d'échantillon de 4 ml contenant au total 100 mg du matériel précédent. Les fractions principales sont recueillies selon le profil en densité optique à 240nm. Ces fractions -sont concentrées et lavées sur membrane PM₁₀ (Amicon) par de l'eau rétro-osmosée contenant 4% de butanol, puis lyophilisées. Une fois pesée, chaque fraction est conservée à -20°C. Seule la fraction 1 de cette étape contient la majorité des molécules reconnues par les anticorps des cobayes immunisés avec des bacilles vivants; elle est soumise à l'étape suivante de séparation.

### 5) Colonne phase inverse:

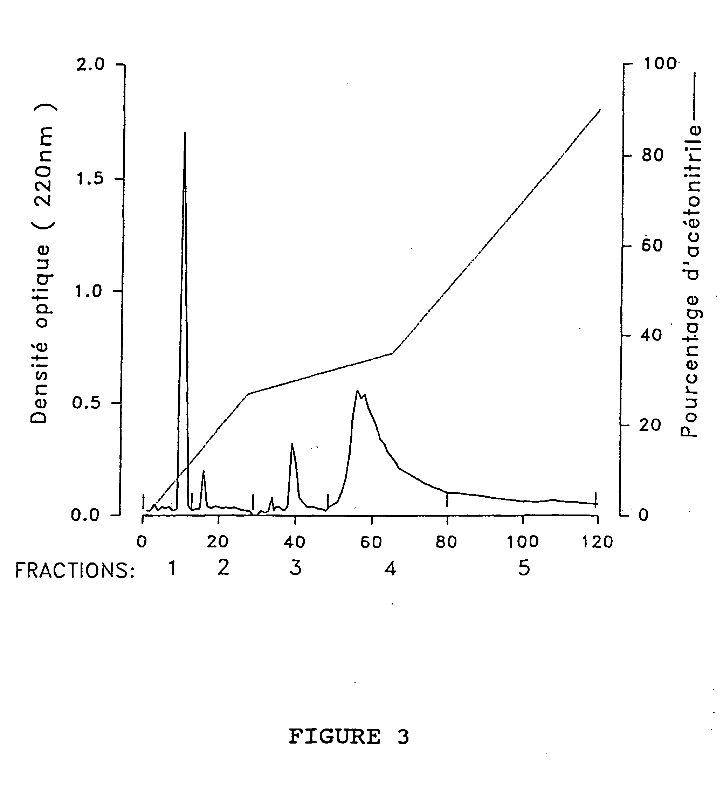

Une colonne RP 300 C₈ 10 µm de 4,6 x 250 mm (Aquapore Brownlee lab.) est équilibrée avec un tampon acétate d'ammonium (NH₄COO CH₃ 20 mM) filtré sur 0,22 µm avec un débit de 2 ml/mn sous une pression maximum de 115 bars. Le tampon d'élution contenant 90% d'acétonitrile est appliqué selon le profil indiqué sur la figure 3 après injection d'un échantillon de 10 mg sous un volume de 1 ml. Le profil de densité optique à 220 nm permet de séparer cinq fractions principales qui sont concentrées par évaporation sous vide à 40°C, puis lyophilisées.

### 6) Immunodétection des antigènes:

Des gels dénaturants à 10% de polyacrylamide, 0,1 % de SDS sont préparés selon la technique classique de Laemmli, (Nature 1970, 277: 680-685). Des échantillons contenant entre 10 et 2 µg de matériel, en fonction de l'étape de la purification, sont appliqués dans un tampon contenant 5% de mercaptoéthanol, 3% de SDS et une trace de bleu de bromophénol sous un volume de 10 µl dans chaque piste du gel. Après électrophorèse jusqu'à la limite de migration du bleu, les molécules présentes dans les échantillons sont transférées sur une feuille de PVDF (Millipore) en appliquant un champ électrique modéré durant une nuit (Harlow et Lane, Antibodies, A Laboratory manual. Cold Spring Harbor Laboratory (eds) 1988).

Une coloration de la feuille de PVDF par une solution de bleu de Coomassie durant moins d'une minute , suivie d'une décoloration, permet le repérage des marqueurs de poids moléculaire dont les contours sont entourés d'un trait de crayon. Après décoloration totale, la feuille est lavée durant 30 mn à la température du laboratoire par du PBS + Triton X100 3%, puis trois fois 5 mn avec du PBS seul. La feuille est alors saturée avec du PBS contenant 5% de lait écrémé en poudre durant 1 h à 37°C, puis lavée avec du PBS + Tween 20 (0,2 %) trois fois.

Une incubation est effectuée avec les immunsérums dilués au 1/20è dans le tampon PBS + Tween 20 (0,2 %) + lait en poudre 5% durant 1 h 30 à 37°C avec des agitations périodiques. Puis trois lavages en PBS + Tween sont opérés avant l'incubation avec les anticorps anti-immunoglobulines marqués avec la phosphatase alcaline. Les anticorps anti-immunoglobulines humains et anti-immunoglobulines de cobayes, marqués à la phosphatase (Biosys) sont utilisés à la dilution finale de 1/2500 en PBS + Tween 20 (0,2 %) + lait (5%). Après 1 h 30 d'incubation à 37°C, les feuilles de PVDF sont lavées trois fois en PBS + Tween, puis incubées à la température du laboratoire durant 5 à 10 mn, dans le tampon de révélation contenant BCIP et NBT (Harlow et Lane, précédemment cités). La réaction est arrêtée et après séchage des feuilles celles-ci sont photographiées.

### 7) Composition en acides aminés:

Une analyse de la composition globale en acides aminés est effectuée pour chaque fraction chromatographique dans l'unité de Chimie Organique de l'Institut Pasteur. Un analyseur Beckman LS 6300 est utilisé.

La composition - globale exprimée en fréquence des acides aminés des protéines de 45-47 kD est la suivante:
ASN/ASP: 10,4%, THR: 5,7%; SER:5,6%; GLN/GLU: 6,3%; GLY:7,1%; ALA: 19,3%; VAL: 6,2%; ILE:2,2%; LEU: 4,4%; TYR: 2,2%; PHE: 2,4%; LYS : 2,7 %; ARG: 2,7%; PRO: 20,9%.

### EXEMPLE 2:

### Détermination de la spécificité immunologique des protéines et fractions protéiques de M.tuberculosis et isolement des antigènes reconnus par les anticorps des cobayes immunisés avec des bacilles vivants.

Des groupes de 12 à 15 cobayes (femelles Hartley de 250 à 300 g au début de l'expérience) ont reçu soit des mycobactéries vivantes (2 x 10⁷ unités viables de BCG en deux injections intradermiques dans 0,1 ml de solution saline), soit 2 mg de mycobactéries de la même souche tuées par la chaleur (120°C, 30 mn) en intramusculaire dans 0,5 ml d'une émulsion solution saline dans l'adjuvant de Freund incomplet (1/1). Les sérums de différents groupes de cobayes ont été prélevés 7 à 12 mois après l'immunisation, filtrés (0,22 µm) puis distribués en petits volumes qui sont congelés et conservés à -20°C. Des essais de plusieurs groupes d'immunsérums ont été effectués (5 après immunisation avec des bactéries vivantes et 6 après immunisation avec des bactéries tuées). Les résultats rapportés ont été obtenus avec un groupe de sérums représentatifs de chaque type d'immunisation; les différences entre groupes sont minimes pour le même schéma d'immunisation.

### 1) Etape de séparation sur la colonne échangeuse d'ions à basse pression.

Le milieu de culture (lavé et concentré sur membrane Amicon PM10 puis lyophilisé) est ultracentrifugé puis appliqué sur la colonne échangeuse d'ions à basse pression. Deux fractions l'une non retenue par la colonne et l'autre éluée par une solution tamponnée à haute molarité sont recueillies, lavées et concentrées sur membrane Amicon PM10 puis lyophilisées.

Chaque fraction (10µg) est placée sur une piste d'un gel SDS, puis, après la séquence d'électrophorèse, transfert sur membrane PVDF et immunodétection, les fractions contenant les molécules majoritaires réagissant avec les différents sérums sont repérées.

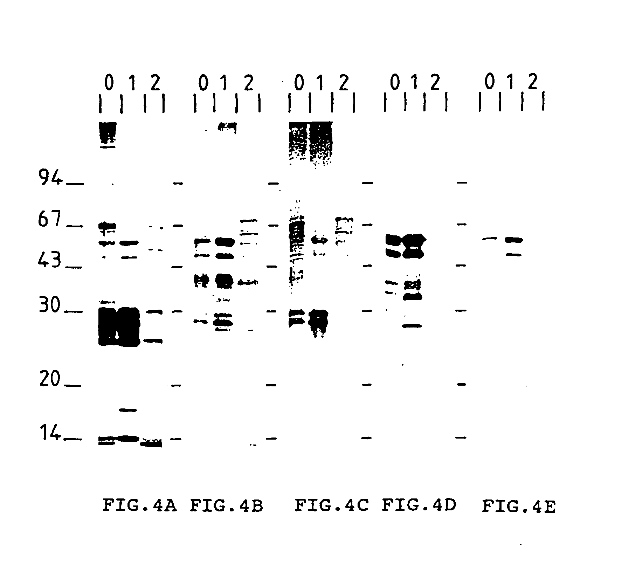

La figure 4 montre des immuno-empreintes de gels identiques révélées avec un colorant de protéines transférées (Aurodye-Amersham) (4A) ou des sérums de cobayes immunisés avec des bacilles vivants (4B) ou morts (4C). Les immuno-empreintes 4D et 4F ont révélées respectivement un sérum de lapin dirigé contre des molécules identiques de BCG (Infection and Immunity, 1993 61 742-750) et le surnageant de l'hybridome I-1081 producteur d'un anticorps monoclonal, déposé auprès de la Collection Nationale de Cultures de Microorganismes de l'Institut Pasteur (CNCM). Seule la fraction non retenue par la colonne contient des molécules de 45/47 kDa reconnues par les sérums de cobayes immunisés avec des bacilles vivants ou reconnues par le surnageant de l'hybridome cité précédemment.

### 2) Etape de filtration moléculaire sur Si 300.

La fraction non retenue de l'étape précédente est injectée sous un volume d'échantillon de 10 ml contenant 500 mg de matériel sur la colonne Si 300. Les fractions 1 à 5 sont séparées selon le profil indiqué sur la figure 1, regroupées pour les injections successives, puis lavées, concentrées et lyophilisées.

Chaque fraction (10 µg) est placée sur une piste d'un gel SDS; puis, après la séquence d'électrophorèse, transfert sur membrane PVDF et immunodétection, les fractions contenant les protéines majoritaires réagissant avec les différents sérums sont repérées.

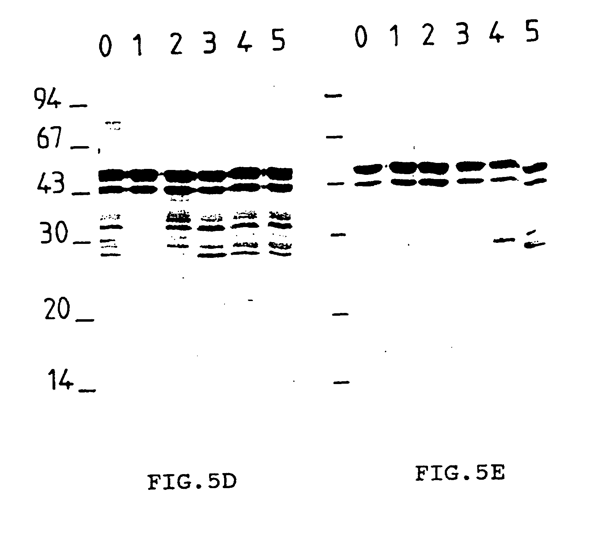

La figure 5 montre des immuno-empreintes de gels identiques révélées avec une coloration des protéines (Aurodyne-Amersham) ou avec des sérums de cobayes immunisés avec des bacilles vivants (5B) ou avec des bacilles morts (5C). Les immuno-empreintes 5D et 5E ont été révélées avec respectivement un sérum de lapin dirigé contre ces molécules purifiées à partir de BCG et avec l'anticorps monoclonal I-1081.

Deux antigènes de 45 et 47 kD présents dans la fraction 1 sont reconnus essentiellement par les anticorps des animaux immunisés avec des bacilles vivants ou avec le sérum polyclonal de lapin ou avec l'anticorps monoclonal. Cette fraction est sélectionnée pour la seconde étape de purification.

### 3) Etape sur colonne échangeuse d'ions

Un échantillon de 100 mg de la fraction précédente est chargé sur une colonne DEAE-TSK préparative et élué par un gradient de NaCl. Le profil à 220 nm des molécules éluées définit trois fractions principales (figure 2). Après regroupement, chaque fraction obtenue par les injections successives du matériel est lavée, concentrée et lyophilisée.

Après électrophorèse sur gel SDS de 5 µg de chacune des fractions précédentes, les immuno-empreintes sur feuille PVDF sont révélées par le colorant de protéines (Aurodyne) (Fig. 6A), par les sérums de cobayes immunisés avec des bacilles vivants (fig. 6B) ou morts- (Fig.6C), le sérum de lapin (Fig.6D) ou l'anticorps monoclonal (Fig. 6E). La fraction 1-DEAE ne contient que quelques antigènes reconnus par les anticorps des animaux immunisés avec des bacilles morts. Par contre, cette même fraction 1-DEAE contient un doublet à 45/47 kD fortement reconnu par les anticorps des cobayes immunisés avec des bacilles vivants, ainsi que le sérum de lapin ou l'anticorps monoclonal. Cette fraction 1-DEAE est choisie pour l'étape suivante de purification.

### 4) Etape sur colonne phase-inverse

Une colonne RP 300 10 µm, équilibrée avec le tampon acétate d'ammonium (20 mM), reçoit un échantillon de 1 ml contenant 5 à 10 mg maximum de la fraction 1-DEAE précédente. L'élution avec un gradient d'acétonitrile de 0 à 90% selon le schéma de la figure 3 permet de récupérer cinq fractions principales. Ces fractions sont concentrées par évaporation sous vide à 40°C pour éliminer la majorité de l'acétonitrile, puis lyophilisées.

La fraction 4 (gradient de 30 à 50% d'acétonitrile) contient la majorité des molécules reconnues par les anticorps des animaux immunisés avec des bacilles vivants, ou par les anticorps présents dans le sérum de lapin ou par l'anticorps monoclonal et essentiellement ces molécules, d'après la coloration des protéines par Aurodyne (Fig.6).

### EXEMPLE 3:

### Clonage et expression de protéines de 45/47 kD de Mycobactérium tuberculosis dans Mycobacterium smegmatis et Escherichia coli.

### 1) Matériels et méthodes.

### 1.1 Souches bactériennes et conditions de croissance, préparations de surnageants et d'extraits bactériens.

M.bovis BCG (souche 1173P₂) a été cultivé en milieu synthétique de Sauton 7 jours à 37°C, puis le surnageant est filtré à travers une membrane de 0,22 µm. Ces surnageants sont conservés bruts en présence de butanol à 4% ou concentrés sur membrane Amicon-PM 10 et lyophilisés.

M. smegmatis mc² 155 (Snapper et al.1990, Molecular Microbiol., 4, 1911-1919) a été cultivé en milieu liquide 7H9 + OADC 7 jours à 37°C. Chaque clone de M.smegmatis mc² 155 transformé par les cosmides de la banque pYUB18: M.tuberculosis a été cultivé en présence de kanamycine à 25 mg/ml. Les cultures ont été ensuite centrifugées 15 mn à 5000 tpm, les surnageants de culture séparés et conservés à 4°C en présence de butanol à 4%. Ces préparations sont utilisées lors de tests ELISA dans lesquels la composition des milieux de culture n'interfère pas. Lorsque les surnageants de culture de clones sont analysés en gel SDS-PAGE, ceux-ci sont cultivés en milieu synthétique de Sauton 7 jours à 37°C, les surnageants de culture filtrés à travers une membrane de 0,22 µm puis concentrés sur membrane Amicon-PM10 et lyophilisés.

Les souches de E.coli NM554 et XL 1-Blue ont été cultivées en milieu Luria-Bertani (LB) solide ou liquide à 37°C. Les clones de E.coli XL 1-Blue, transformés par le plasmide pUC18, ont été cultivés en présence d'ampicilline à 25 µg/ml.

Les lysats de culture bactérienne de E.coli XL1-Blue et de chaque clone transformé par les plasmides recombinants pUC18: M.tuberculosis ont été préparés par une série de congélations/décongélations rapides de -70°C à + 60°C de bactéries obtenues après une culture d'une nuit (16h). Les lysats sont centrifugés, les surnageants séparés et conservés à - 20°C. Un dosage des protéines de ces préparations a été effectué par la technique BCA (Pierce).

### 1.2 Vecteurs de clonage

La banque génomique de M.tuberculosis utilisée (Jacobs et al. 1991 précédemment cités) a été fournie par électroporation dans M.smegmatis mc² 155 par Stewart Cole. Le demandeur a disposé de 400 clones recombinants.

La banque a été construite dans un cosmide, vecteur navette pYUB18. Celui-ci dérive du plasmide pYUB12 (Snapper et al. Proc. Natl. Acad. Sci, USA 1988, 85: 6987-6991) dans lequel a été insérée la séquence Cos du bactériophage lambda permettant une amplification et une bonne conservation des cosmides recombinants de la banque sous forme de lysats de phages. Cette banque a été construite de la manière suivante: l'ADN génomique de M.tuberculosis souche H37Rv a été digéré partiellement par l'enzyme Sau 3a, dans des conditions permettant d'obtenir un maximum de fragments de 35 kb à 45 kb. Ces fragments ont été purifiés puis ligaturés dans pYUB18, digérés par l'endonucléase de restriction BamHI et déphosphorylés.

Le vecteur plasmidique pUC18 (Yanisch-Perron et al. Gene, 1985, 33: 103-119) a été utilisé pour le sous-clonage dans E.coli XL-Blue. Ce plasmide multicopie porte un fragment d'ADN dérivé de l'opéron lac de E.coli qui code pour un fragment amino-terminal de la bêta-galactosidase. Ce fragment inductible par l'isopropyl bêta-D-thiogalactopyranoside (IPTG) est capable d'établir l'alpha-complémentation avec la forme de la bêta-galactosidase défective codée par la souche hôte E.coli XL1-Blue. Ainsi l'insertion d'ADN étranger va induire une abolition de l'alpha-complémentation. Les plasmides recombinants peuvent être repérés lorsqu'ils sont transformés dans la souche hôte par la - couleur blanche des colonies, comparativement à la couleur bleu des colonies lorsque les bactéries sont transformées par le plasmide pUC18. Ce criblage s'effectue en présence d'IPTG et du substrat de l'enzyme le X-Gal.

### 1.3 Techniques de biologie moléculaire

### 1.3.1 Extraction de cosmides de M.smegmatis mc2 155

Les extractions de cosmides recombinants pYUB18:M.tuberculosis ont été effectuées selon la technique de lyse alcaline adaptée à M.smegmatis (Jacobs et al.1991, précédemment cité) avec quelques modifications. Les bactéries sont récupérées au cinquième jour de culture (fin de la phase exponentielle), centrifugées 10 mn à 5000 tours. Le culot de bactéries (3 ml) est resuspendu dans 5 ml de solution A(50 mM glucose, 25 mM trisHCl pH 8, 10 mM EDTA, lysosyme 10 mg/ml) et incubé à 37°C pendant 20 mn. Deux volumes (10 ml) de solution B (O,2 N NaOH, 1% SDS) sont ensuite ajoutés et mélangés par inversion. Le mélange est incubé 30 mn à 65°C, puis 15 mn à 4°C. Enfin 1,5 volume (7,5 ml) de solution C (5 M acétate de potassium, acide acétique 11,5%) est ajouté et mélangé par inversion. Le mélange est incubé 30 mn à 4°C. La préparation est ensuite centrifugée 15 mn à 13000 tours à 4°C, le surnageant récupéré, mesuré et additionné à un même volume de phénol/chloroforme 50/50.

Après extraction le tube est centrifugé à 4000 tours pendant 10 mn. La phase aqueuse est transférée dans un tube propre et additionnée d'un double volume d'éthanol conservé à -20°C. Après retournement, celui-ci est conservé au moins 1 heure à -20°C, puis centrifugé 30 mn à 12000 tours. le culot est enfin lavé avec un volume d'éthanol à 70% conservé à -20°C et séché au Speed-Vac 5 mn. Le culot sec est repris dans 500µl d'eau stérile et conservé à -20°C.

### 1.3.2 Extraction et purification de plasmides de E.coli

Les extractions rapides des cosmides pYUB18 et plasmides pUC18 recombinants ont été effectuées par la technique de lyse alcaline (Birnboim et al.; Nucleic Acids Res.1979, 7:1513).

Les cosmides et plasmides recombinants intéressants ont été purifiés après une étape de lyse alcaline par ultracentrifugation en gradient de chlorure de césium en présence de bromure d'éthidium (Maniatis et coll, Cold Spring Harbor , New York: Cold Spring Harbor Laboratory Press 1982).

### 1.3.3. Techniques de transformation

### Méthode chimique au chlorure de calcium.

Cette technique classique a été utilisée pour transformer E.coli XLl-Blue par des plasmides pUC18 recombinants. Les bactéries compétentes sont tout d'abord préparées: 20 ml de milieu 2YT sont ensemencés avec une préculture d'une nuit au 1/100. Les bactéries sont mises en culture agitée pendant 2 heures à 37°C jusqu'à DO = 0,6, puis centrifugées 10 mn à 4000 tours à 4°C. Le culot est repris dans 8 ml de CaCl₂, 100 mM, gardé 15 mn sur glace fondante, puis centrifugé à nouveau 10 mn à 4000 tours à 4°C. Le culot est enfin repris dans 1,6 ml de CaCl₂ 100 mM gardé sur glace fondante à 30 mn.

Les bactéries compétentes ainsi préparées sont utilisées fraîchement pour les transformations ou peuvent être conservées quelques jours à 4°C. Au moment de la transformation 200 µl de bactéries compétentes sont mélangés à 2 µl d'ADN. Le mélange est conservé 45 mn sur glace fondante, puis est soumis à un choc thermique de 2 mn à 42°C. 800 µl de milieu 2YT sont ajoutés, puis la préparation est incubée une heure à 37°C sous agitation, puis étalée sur boîtes ML-ampicilline à raison de 50 µl à 200 µl par boîte. Le lendemain les colonies sont comptées et l'efficacité de transformation calculée.

### - Méthode physique d'électroporation.

Cette technique est utilisée pour transformer E.coli par des vecteurs de grande taille: E.coli souche NM554 a été électroporée par des cosmides pYUB18 recombinants de taille supérieure à 50 kb. Les bactéries compétentes ont été préparées fraichement: 200 ml de milieu 2YT sont ensemencés par une dilution au 1/100 d'une préculture d'une nuit; les bactéries sont cultivées 3 heures à 37°C, puis centrifugées à 6000 tours pendant 10 mn. Le culot est repris dans 10 ml d'eau stérile à 4°C, puis dans 190 ml d'eau stérile à 4°C. Les bactéries sont centrifugées à nouveau à 6000 tours 10 mn et relavées dans 10 ml d'eau stérile à 4°C. Enfin le culot est repris dans 400 µl de glycérol à 10 %.

L'électroporation a été effectuée sur un appareil Bio-Rad Gene Pulser. 100 µl de bactéries sont mélangés à 1 à 4 µl d'ADN dans une cuve de 0,4 mm. Le mélange est soumis au choc électrique (2500 volts, 25 µF). Puis rapidement 1 ml de milieu 2 YT est ajouté dans la cuve. L'ensemble est transféré dans un tube et incubé 1 heure à 37°C sous agitation. Après incubation, la culture est étalée sur boîtes ML-ampicilline, à raison de 50µl à 200 µl par boîte. Le lendemain, les colonies sont comptées et l'efficacité de transformation calculée.

### 1.3.4 Clonage de fragments de digestion enzymatique

L'ADN à cloner a été digéré par une endonucléase de restriction BamHI. De la même manière le plasmide pUC18 a-été digéré. Les fragments issus d'un cosmide pYUB18 recombinant d'intérêt ont été ligaturés dans le plasmide vecteur par l'activité de l'enzyme T4 DNA ligase (Amersham). La ligation a été effectuée dans un volume de 20µl à 16°C pendant une nuit. Tout le mélange de ligation a été utilisé pour la transformation dans E.coli XL1-Blue. Après l'expression phénotypique toutes les bactéries sont étalées sur boîtes ML-ampicilline à 25 µg/ml, IPTG, X-Gal. Les clones recombinants ne permettant plus l'alpha-complémentation ont été repérés par la couleur blanche des colonies.

Les clones recombinants ont été étudiés après purification par clonage. L'ADN plasmidique a été extrait par lyse alcaline puis analysé sur gel d'agarose à 0,8 % avant ou après digestion par l'endonucléase de restriction BamH I.

### 1.3.5 Construction d'une carte de restriction

Les plasmides recombinants pLA34 et pLA4, contenant un insert de 3 kb BamH I-BamH I cloné dans les deux orientations, ont été digérés par les différentes endonucléases de restriction présentant un site dans le lieur multisite (polylinker) de pUC18. Des digestions simples et doubles à l'aide des endonucléases de restriction, BamH I, Hind III, Sph I, Xba I, Sal I, Kpn I, EcoR I, Sma I ont été effectuées puis analysées en gel d'agarose à 0,8 %. Après coloration de l'ADN au bromure d'éthidium la taille des différents fragments est déterminée en fonction de leur distance de migration par rapport à des marqueurs (un témoin interne de laboratoire, le plasmide pKN digéré par Pvu II).

### 1.4. Méthodes de détection des protéines.

### 1.4.1. Technique ELISA

Un test ELISA en compétition a été utilisé pour mesurer la concentration des protéines 45/47 kDa présentes dans différentes préparations issues de cultures bactériennes, à l'aide d'un sérum polyclonal (Romain et al., 1993, précédemment cités).

Ce sérum polyclonal de lapin a été obtenu contre les protéines 45/47 kDa par une technique classique d'immunisation: injection de 50 µg de protéines purifiées en adjuvant de Freund incomplet et de 25 µg un mois plus tard.

Les puits d'une première microplaque sont recouverts soit par les protéines purifiées en solution à la concentration de 1 µg/ml en tampon carbonate soit par un surnageant de Mycobacterium bovis BCG de 15 jours à la concentration de 10 µg/ml. La fixation des antigènes est effectuée pendant une heure à 37°C, puis la microplaque est lavée 5 fois au PBS. Dans une seconde incubation les puits sont saturés par une solution de PBS gélatine 0,5 %, butanol 4%, pendant une heure à 37°C. La microplaque est ensuite lavée 5 fois au PBS-Tween 0,1 %.

Le test est effectué de la manière suivante:

Incubation dans une seconde microplaque de 50 µl du surnageant à analyser à des dilutions différentes (pur, 1/2, 1/4, 1/8, etc....) dans du PBS-Tween 0,1 % , gélatine 0,25%, butanol 4%, et de 50µl du sérum de lapin préparé à la dilution 1/4000 dans du PBS-tween 0,1 %, gélatine 0,25 %, butanol 4%, une heure à 37°C, puis transfert sur la première microplaque du mélange et incubation une heure à 37°C. La microplaque est ensuite lavée 10 fois au PBS-tween 0,1 %. Enfin, un anticorps conjugué anti IgG H + L anti-lapin (BioSys) marqué à la phosphatase alcaline, préparé à la dilution du 1/4000 dans du PBS-tween 0,1 %, gélatine 0,25%, butanol 4%, a été incubé une heure à 37°C. La microplaque est lavée 10 fois au PBS-tween 0,1%.

Le substrat de l'enzyme, le para-nitro-phényl-phosphate (pNPP) est enfin incubé à la concentration de 40 mg/24ml en tampon NaHCO₃, MgCl₂ pH 9,6 pendant une heure ou une nuit. Les DO sont lues à 414 nm et 690 nm par le lecteur Titerteck Twinreader.

### 1.4.2. Technique d'immuno-empreinte

La technique classique d'électrophorèse en gel dénaturant SDS-PAGE a été utilisée ( Laemmli, Nature 1970, 277:680-685), suivie d'un électrotransfert sur membrane de PVDF (Towbin et al. Proc. Natl. Acad. Sci. USA 1979, 76:4350-4354, Pluskal et al. Biotechniques 1986, 4: 272-283).

Les échantillons analysés sur gel sont quantitativement mesurés: en µg de lyophilisat pour les surnageants de M.smegmatis (5 µg sont déposés), en µg de protéines pour les lysats de E.coli (25 µg sont déposés).

Les protéines purifiées de M.bovis BCG sont déposées sur gel à raison de 0,25 µg de protéines par piste.

Les protéines transférées sur membrane sont révélées par le sérum polyclonal de lapin à une dilution au 1/500è pour les protéines exprimées dans les mycobactéries.

Pour révéler les protéines recombinantes dans E.coli, ces anticorps polyclonaux ont été purifiés sur une colonne DEAE (Trisacryl^{D}), puis les immunoglobulines obtenues absorbées sur un lysat de E.coli immobilisé sur colonne Sépharose-4B activé par le bromure de cyanogène (Pharmacia) (Maniatis et coll, 1982). Les anticorps non retenus sont conservés en pool à 4°C et utilisés pour révéler les protéines transférées sur membrane à une dilution au 1/100è.

Un conjugué anti-Ig H + L (Bio-Sys), spécifique d'espèce, marqué par la phosphatase alcaline, est utilisé pour révéler les anticorps précédents à une dilution au 1/3000. Enfin l'activité phosphatase alcaline est révélée par deux substrats artificiels chromogènes: le bleu de tétrazolium et le 5-bromo 4 chloro 3-indolyl phosphate.

### 1.5. Séquençage de l'ADN:

Le séquençage nucléotidique a été effectué par l'utilisation d'un ensemble de clones obtenus par différentes délétions à partir des deux clones pLA34 et pLA4. Les délétions ont été choisies en fonction de la carte de restriction établie.

Le séquençage a été effectué à partir des matrices d'ADN plasmidique double-brin. La technique de Sanger a été appliquée par l'utilisation du kit T7 Sequencing (Pharmacia) et du ³⁵S ATP.

La séquence a été obtenue à l'aide de différents clones délétés et des amorces universelles (Direct and Reverse Primers) du plasmide pUC18, puis d'oligonucléotides synthétiques.

Les séquences ont été établies sur les deux brins complémentaires.

Les zones de compression dues au pourcentage élevé en GC de l'ADN génomique de M.tuberculosis (65%) ont été séquencées à l'aide du kit T7 Deaza G/A Sequencing (Pharmacia) contenant un analogue chimique du dGTP, le 7-Deaza dGTP.

### 1.6. Analyse des séquences:

Les comparaisons et assemblages des séquences contiguës obtenues ont été effectués à l'aide du programme STADEN sur Unix. Les homologies de séquences recherchées parmi les séquences des banques de données EMBL et Gen-Bank ont été faites en utilisant les programmes FASTA et T-FASTA de GCG.

### 2) Résultats

### 2.1 Clonage et expression des protéines de 45/47 kDa de M.tuberculosis dans M.smegmatis.

### 2.1.1. Criblage d'une banque génomique d'expression de M.tuberculosis dans M.smegmatis.

La banque génomique utilisée (Jacobs et al., 1991 précédemment cités) a été construite par clonage de fragments de 40 kb issus d'une digestion génomique partielle par l'endonucléase de restriction Sau 3a dans le vecteur cosmidique pYUB18. La taille du génome, estimée par électrophorèse en champs pulsé à 4200 kb est donc contenue dans environ 100 à 150 clones.

Un test ELISA en compétition permettant de doser les protéines en milieu liquide a été utilisé (Romain et al., 1993, précédemment cités). Il permet de détecter et de définir une quantité de protéines de 45/47 kDa dans les surnageants de culture de M.bovis BCG de 7 jours (figure 8).

Ce test présente des avantages: une bonne sensibilité, c'est-à-dire la capacité de détecter une quantité de l'ordre de 1 ng/ml de protéines en milieu liquide à l'aide d'un sérum polyclonal dilué au 1/8000è (Romain et al. 1993, précédemment cités) et une facilité de mise en oeuvre pour cribler une série d'échantillons rapidement.

Une série de 400 clones recombinants pYUB18::M.tuberculosis H37Rv, électroporés dans M. smegmatis, a été criblée.

Pour cela, les différents clones ont été cultivés pendant 7 jours en milieu 7H9 + OADC. Les protéines recombinantes sont recherchées dans ce test en analysant les surnageants obtenus après centrifugation des cultures.

Trois clones ont été trouvés capables d'exprimer des protéines reconnues par les anticorps polyclonaux spécifiques des protéines de 45/47 kDa de M.bovis BCG (figure 8). Lors de ce premier criblage les puits des plaques de microtitration étaient recouverts par un surnageant de culture de M.bovis BCG dans lequel les protéines de 45/47 kDa ont été évaluées à 2% de la masse totale. Les trois clones sélectionnés ont été confirmés dans une seconde expérience dans laquelle les puits des plaques de microtitration étaient recouverts par les protéines de 45/47 kDa purifiées.

### 2.1.2 Analyse génétique des cosmides recombinants sélectionnés.

Dans le but d'étudier les différents cosmides sélectionnés, ceux-ci ont été électroporés dans E.coli NM554 après extraction de l'ADN de M.smegmatis par lyse alcaline modifiée. L'obtention de l'ADN extrachrosomique de mycobactéries est en effet un point difficile dû à la complexité de la paroi difficile à lyser d'une part et au faible nombre de copies des vecteurs qui a été déterminé de 3 à 10 en moyenne par bactérie. Les trois clones transformés dans E.coli NM554 ont été isolés sur boîtes ML-kanamycine, l'ADN cosmidique, extrait par lyse alcaline a été analysé en gel d'agarose à 0,8%.

Les trois clones présentent un ADN de taille supérieure à 50 kb. Une digestion par l'endonucléase de restriction BamH I a été effectuée pour distinguer les profils de ces trois cosmides sélectionnés. Ceux-ci se sont révélés identiques (figure 9). Les profils montrent une bande de 12 kb correspondant au vecteur pYUB18, puis une série de bandes de plus bas poids moléculaire correspondant au fragment de l'ADN cloné (environ 40 kb). Compte-tenu du nombre de bandes obtenues et de leur localisation en gel, on peut estimer que les cosmides isolés étaient identiques.

Différentes digestions du seul cosmide pLA1, par les endonucléases de restriction présentant des sites de coupures plus ou moins fréquents pour un ADN riche en G + C ont été effectuées dans le but de distinguer des fragments de longueurs moyennes et suffisantes pour contenir le ou les gènes de structure des protéines de 45/47 kDa et d'effectuer un sous-clonage de ceux-ci ( figure 10).

### 2.1.3. Expression des protéines de 45/47 kDa de M.tuberculosis dans M.smegmatis.

Le cosmide pLA1, contenant un insert d'environ 40 kb, permet l'expression de protéines recombinantes dans M.smegmatis, détectées dans un surnageant de culture par des anticorps polyclonaux.

Afin de déterminer la taille approximative des protéines exprimées, un surnageant de culture de 7 jours lyophilisé a été analysé en immuno-empreinte. Les protéines recombinantes exprimées dans M.smegmatis présentent deux poids moléculaires de 45/47 kDa apparents identiques à celles exprimées dans M.bovis BCG (figure 11).

Dans une autre expérience, le niveau d'expression de ces protéines recombinantes dans M.smegmatis a été comparé à celui dans M.bovis BCG. Une quantité déterminée de protéines de surnageants lyophilisés est utilisée lors d'un dosage en test ELISA en compétition. Différentes concentrations des surnageants lyophilisés sont mises en présence d'une dilution au 1/8000è du sérum polyclonal de lapin. M.smegmatis recombinant permet l'expression de protéines en quantité environ 5 fois plus importante que M.bovis BCG (figure 12).

Un sous-clonage de cet insert ainsi que l'analyse des protéines recombinantes dans un hôte hétérologue (E.coli) ont été effectués afin de déterminer le nombre de gènes codant pour ces protéines.

### 2.2. Clonage et expression des protéines de 45/47 kDa de M.tuberculosis dans E.coli.

### 2.2.1. Sous-clonage et expression des protéines de 45/47 kDa dans E.coli.

Lorsque pLA1 a été transformé dans un hôte hétérologue E.coli NM554, aucune protéine recombinante n'a été détectée dans les surnageants de culture ou lysats bactériens. Dans le but de favoriser l'expression de ces protéines, un sous-clonage des fragments issus d'une digestion BamH I du cosmide a été effectué dans le plasmide pUC18 (Yanisch-Perron et al. Gene,1985, 33:103,119).

Les plasmides recombinants pUC18::M.tuberculosis transformés dans E.coli XL1-Blue ont été sélectionnés par défaut d'expression de la bêta-galactosidase de la bactérie hôte. L'ADN plasmidique de chaque clone "blanc" d'une série de 36 clones) a été préparé par lyse alcaline et digéré par l'endonucléase de restriction BamH I.

La taille des plasmides obtenus observée en gel d'agarose montre plusieurs profils indiquant que les plasmides recombinants sont différents (figure 13A).

La taille des inserts clonés également observée en gel d'agarose montre des profils de restriction différents (figure 13B). Ces profils présentent tous un fragment de 2,8 kb correspondant au vecteur pUC18 et une série de fragments de tailles différentes correspondant aux inserts clonés.

Tous les fragments de la digestion ont été clonés seuls, par deux ou par trois excepté le fragment de 12 kb qui par sa grande taille est difficilement clonable.

Les 36 clones sélectionnés ont été criblés sur leur capacité à induire une expression de protéines recombinantes dans E.coli XL1-Blue. Cette expérience a été effectuée dans le même test ELISA en compétition que précédemment.

Aucune protéine recombinante n'a été détectée dans les surnageants de culture bactérienne. Par contre, des protéines recombinantes ont été détectées dans les lysats bactériens de clones contenant tous au moins un insert de 3 kb.

Le niveau d'expression des protéines mesuré dans ce test apparaît influencé par la taille des plasmides. Parmi les 36 clones étudiés, 2 clones permettant une expression, le pLA34 et le pLA35, contenant respectivement des inserts de 3kb et 7 kb ont été trouvés. Celle-ci est plus importante pour le pLA34 comme le montrent les résultats du tableau 1 (ci-après).

### 2.2.2. Carte de restriction des clones pLA34 et pLA34-2.

Une carte de restriction du plasmide pLA34 a été établie, situant différents sites de coupure d'endonucléases de restriction courantes, présents dans le lieur multisite (polylinker) de pUC18 (figure 14). Un site unique de restriction EcoR I sépare l'insert de 3 kb en deux fragments de 2 kb et 1 kb.

Un clone, le pLA34-2 présentant un insert de 2 kb BamH I-EcoR I a été construit à partir du précédent par délétion. Celui-ci permet également l'expression de protéines recombinantes détectées dans des lysats bactériens (figure 15).

L'analyse des lysats bactériens en immuno-empreinte montre des protéines de deux poids moléculaires de 45 et 47 kDa, apparemment identiques aux protéines natives exprimées dans M.bovis BCG (figure 16).

### 2.2.3. Analyse de la séquence nucléotidique codant pour les protéines de 45/47 kDa de M.tuberculosis H37Rv:

La séquence nucléotidique complète du gène qui code pour les protéines de 45/47 kDa, la séquence en amont et la séquence déduite en acides aminés sont présentées sur la séquence SEQ ID N° 1 et SEQ ID N° 2. L'unique gène permettant l'expression du doublet de protéines présente 975 paires de bases entre la position 1082 et la position 2056 inclus de la séquence nucléotidique.

Une séquence consensus de fixation au ribosome (Shine Dalgarno) a été repérée en amont du gène.

Le gène présente un haut pourcentage en GC de 69,4 % comparativement au GC de 65% pour M.tuberculosis.

La protéine déduite du gène présente une séquence signal typique avec un site de clivage ANA de la signal peptidase.

Le gène code pour une protéine de 325 aminoacides qui comporte une séquence signal de 39 aminoacides.

Les résultats obtenus par analyse biochimique de la composition en acides aminés des protéines purifiées de M.bovis BCG et de M.tuberculosis comparés à ceux déduits de la séquence protéique sont en bon accord (tableau 2). Ceci permet de conclure sur l'existence d'un seul gène qui permet l'expression de protéines de deux poids moléculaires dans Mycobacterium smegmatis et E.coli.

### 2.2.4. Analyse de la séquence protéique et comparaison de séquences:

Le poids moléculaire calculé à partir de la séquence déduite en acides aminés est de 28,7 kDa.

Le point isoélectrique calculé est de 4,36. Ce dernier résultat se -trouve également en bon accord avec une détermination biochimique du point isoélectrique effectuée sur les protéines purifiées de M.bovis BCG.

La séquence déduite en acides aminés montre un pourcentage en proline et alanine important (21,8% et 19,1%).

La séquence entière montre une homologie avec une protéine récemment décrite de Mycobacterium leprae. Les deux séquences sont comparées sur la figure 17. Le score d'homologie entre les deux protéines est de 65,4%. Cette protéine décrite chez Mycobacterium leprae présente également une séquence signal typique des protéines sécrétées.

Le profil d'hydrophobicité de la protéine déduite de M.tuberculosis, objet de la présente invention (SEQ ID N° 2) a été établi. Il est représenté sur la figure 18.

**TABLEAU 1:**

| Clonage dans pUC18 d'un insert de 3 kb permettant l'expression de protéines recombinantes dans E.coli | | |
|---|---|---|
| Clones pUC18: M.tuberculosis | Taille des inserts | Expression des protéines ELISA |
| N° 34 | 3 kb | + + |
| N° 35 | 3 kb + 4 kb | + |
| N° 4 | 3 kb | - |
| n° 17 | 3 kb + 4 kb + 1,7 kb | - |

**TABLEAU 2**

| Composition en acides aminés des protéines de M.tuberculosis et M.bovis BCG de 45/47 kDa et de M.leprae de 27/32 kDa | | | | |
|---|---|---|---|---|
| | Séquence déduite | | Analyse chimique* | |
| | (% en moles) | | (% en moles) | |
| Résidu | M.leprae | M.tuber | M.tuber | M.bovis |
| A = Ala | 13.3 | 18.5 | 19.2 | BCG 19.2 |
| B = Asx | - | - | 10.4 | 10.6 |
| C = Cys | 0.4 | 0 | < 0.5 | < 0.5 |
| D = Asp | 4.8 | 5.2 | - | - |
| E = Glu | 4.8 | 3.1 | - | - |
| F = Phe | 2.0 | 2.5 | 2.4 | 2.2 |
| G = Gly | 8.0 | 7.0 | 7.1 | 7.4 |
| H = His | 0.8 | 0.3 | 0.4 | 0.4 |
| I = Ile | 5.2 | 2.5 | 2.2 | 2.3 |
| K = Lys | 2.8 | 2.5 | 2.7 | 2.9 |
| L = Leu | 6.8 | 4.2 | 4.4 | 4.7 |
| M = Met | 0.8 - | 0.7 | 0.5 | 0.5 |
| N = Asn | 4.0 | 4.5 | - | - |
| P = Pro | 13.3 | 21.7 | 20.9 | 21.9 |
| Q = Gln | 3.2 | 2.8 | - | - |
| R = Arg | 2.8 | 2.8 | 2.7 | 2.5 |
| S = Ser | 9.6 | 5.9 | 5.6 | 5.0 |
| T = Thr | 4.8 | 6.3 | 5.7 | 5.4 |
| V = Val | 8.0 | 5.9 | 6.2 | 5.8 |
| W = Trp | 1.2 | 1.4 | N.D. | N.D. |
| Y = Tyr | 2.8 | 2.1 | 2.2 | 2.2 |
| Z = Glx | - | - | 6.3 | 6.0 |

| | | | | |
|---|---|---|---|---|
| * Asx = Asp + Asn Glx = Glu + Gln | | | | |

## Revendications

1. Protéine de séquence SEQ ID N°3 suivante :

2. Protéine selon la revendication 1 **caractérisée en ce qu'**elle présente la séquence SEQ ID N° 2 suivante:

3. Protéine hybride comprenant une des séquences SEQ ID N°2 ou SEQ ID N°3 selon l'une des revendications 1 et 2 et une séquence d'un peptide ou d'une protéine susceptible d'induire une réponse immunitaire.

4. Protéine selon la revendication 3, **caractérisée en ce que** la réponse immunitaire est une réponse humorale et/ou une réponse cellulaire.

5. Protéine selon l'une des revendications 3 et 4 **caractérisée en ce que** le peptide bu la protéine est une partie, en particulier un épitope, de la toxine diphtérique, de la toxine tétanique, de l'antigène HBS du virus HBV, ou de l'antigène VP1 du virus de la poliomyélite ou autre toxine ou antigène viral.

6. Oligonucléotide codant pour une protéine selon l'une des revendications 1 à 5.

7. Oligonucléotide selon la revendication 6 **caractérisé en ce qu'**il présente au moins une partie de la séquence SEQ ID N°1 suivante:

8. Microorganisme transformé avec un oligonucléotide selon la revendication 6 ou 7, ledit microorganisme produisant une protéine selon l'une des revendications 1 à 5.

9. Microorganisme selon la revendication 8, **caractérise en ce que** ladite protéine est présente au moins en partie à sa surface.

10. Microorganisme selon la revendication 9, **caractérisé en ce qu'**il est une bactérie.

11. Microorganisme selon l'une des revendications 8 à 10, **caractérisé en ce qu'**il est une mycobactérie, en particulier M.bovis BCG.

12. Composition pharmaceutique comprenant une quantité efficace d'une protéine ou d'un microorganisme selon l'une des revendications 1 à 5 et 8 à 11 en association avec des diluants ou des excipients pharmaceutiquement compatibles.

13. Médicament ou vaccin comprenant une protéine ou un microorganisme selon l'une des revendications 1 à 5 et 8 à 11.

14. Procédé de détection in vitro d'anticorps spécifiques de la tuberculose dans lequel un fluide biologique, susceptible de contenir lendits anticorps, est mis en contact avec une protéine selon l'une des revendications 1 à 5.

15. Procédé selon la revendication 14, **caractérisé en ce que** lesdites protéines sont fixées sur un support.

16. Trousse de détection pour la mise en oeuvre du procédé selon l'une des revendications 14 et 15 comprenant au moins une préparation de protéine selon l'une des revendications 1 à 5 et des solutions tamponnées pour la mise en oeuvre du procédé.

17. Trousse selon la revendication 16 **caractérisée en ce qu'**elle comprend un réactif mettant en évidence le complexe anticorps-protéine formé.

## Patentansprüche

1. Protein mit der folgenden Sequenz SEQ ID Nr. 3:

2. Protein gemäß Anspruch 1, **dadurch gekennzeichnet, dass** es die folgende Sequenz SEQ ID Nr. 2 aufweist:

3. Hybridprotein, das die Sequenzen SEQ ID Nr. 2 oder SEQ ID Nr. 3 gemäß einem der Ansprüche 1 und 2 sowie eine Sequenz eines Peptids oder eines Proteins, das eine Immunantwort zu induzieren vermag, umfasst.

4. Protein gemäß Anspruch 3, **dadurch gekennzeichnet, dass** die Immunantwort eine humorale Antwort und/oder eine zelluläre Antwort ist.

5. Protein gemäß einem der Ansprüche 3 und 4, **dadurch gekennzeichnet, dass** das Peptid oder Protein ein Teil, insbesondere ein Epitop, des Diphtherietoxins, des Tetanustoxins, des HBS-Antigens des Virus HBV oder des Antigens VP1 des Poliomyelitisvirus oder eines anderen viralen Toxins oder Antigens ist.

6. Oligonucleotid, das für ein Protein gemäß einem der Ansprüche 1 bis 5 codiert.

7. Oligonucleotid gemäß Anspruch 6, **dadurch gekennzeichnet, dass** es wenigstens einen Teil der folgenden Sequenz SEQ ID Nr. 1 aufweist:

8. Mikroorganismus, der mit einem Oligonucleotid gemäß einem der Ansprüche 6 und 7 transformiert ist, wobei der Mikroorganismus ein Protein gemäß einem der Ansprüche 1 bis 5 erzeugt.

9. Mikroorganismus gemäß Anspruch 8, **dadurch gekennzeichnet, dass** das Protein wenigstens auf einem Teil seiner Oberfläche vorhanden ist.

10. Mikroorganismus gemäß Anspruch 9, **dadurch gekennzeichnet, dass** es ein Bakterium ist.

11. Mikroorganismus gemäß einem der Ansprüche 8 bis 10, **dadurch gekennzeichnet, dass** es sich dabei um ein Mycobakterium, insbesondere *M*. *bovis* BCG, handelt.

12. Pharmazeutische Zusammensetzung, die eine wirksame Menge eines Proteins oder eines Mikroorganismus gemäß einem der Ansprüche 1 bis 5 und 8 bis 11 zusammen mit pharmazeutisch verträglichen Verdünnungsmitteln oder Trägersubstanzen umfasst.

13. Medikament oder Impfstoff, das bzw. der ein Protein oder einen Mikroorganismus gemäß einem der Ansprüche 1 bis 5 und 8 bis 11 umfasst.

14. In-vitro-Nachweisverfahren für Antikörper, die spezifisch für Tuberkulose sind, wobei eine biologische Flüssigkeit, die diese Antikörper enthalten könnte, mit einem Protein gemäß einem der Ansprüche 1 bis 5 in Kontakt gebracht wird.

15. Verfahren gemäß Anspruch 14, **dadurch gekennzeichnet, dass** die Proteine auf einem Träger fixiert sind.

16. Nachweiskit zur Durchführung des Verfahrens gemäß einem der Ansprüche 14 und 15, der wenigstens ein Proteinpräparat gemäß einem der Ansprüche 1 bis 5 und gepufferte Lösungen für die Durchführung des Verfahrens umfasst.

17. Kit gemäß Anspruch 16, **dadurch gekennzeichnet, dass** er ein Reagens umfasst, das den gebildeten Antikörper-Protein-Komplex nachweisen kann.

## Claims

1. Protein having the sequence SEQ ID N°3 following :

2. Protein according to claim 1 **characterized in that** it has the sequence SEQ ID N°2 following:

3. Hybrid protein comprising one of the sequences SEQ ID N°2 or SEQ ID N°3 according to one of claims 1 and 2 and a sequence of a peptide or a protein able to induce an immune response.

4. Protein according to claim 3, **characterized in that** the immune response is a humoral response and/or a cellular response.

5. Protein according to one of claims 3 and 4 **characterized in that** the peptide of the protein is a portion, in particular an epitope, of diphtheria toxin, tetanus toxin, the HBS antigen of the HBV virus, or the VP1 antigen of the poliomyelitis virus or any other viral toxin or antigen.

6. Oligonucleotide coding for a protein according to one of claims 1 to 5.

7. Oligonucleotide according to claim 6 **characterized in that** it has at least a portion of the sequence SEQ ID N°1 following:

8. Microorganism modified with an oligonucleotide according to anyone of claims 6 and 7, said microorganism producing a protein according to one of claims 1 to 5.

9. Microorganism according to claim 8, **characterized in that** said protein is present at least in part on its surface.

10. Microorganism according to claim 9, **characterized in that** it is a bacterium.

11. Microorganism according to one of claims 8 to 10, **characterized in that** it is a mycobacterium, in particular M.bovis BCG.

12. Pharmaceutical composition comprising an effective quantity of a protein or a microorganism according to one of claims 1 to 5 and 8 to 11 in combination with pharmaceutically compatible diluents or adjuvants.

13. Drug or vaccine comprising a protein or a microorganism according to one of claims 1 to 5 and 8 to 11.

14. Method for detecting in vitro specific tuberculosis antibodies, in which a biological fluid, liable to contain said antibodies, is brought into contact with a protein according to one of claims 1 to 5.

15. Method according to claim 14, **characterized in that** said proteins are fixed on a support.

16. Assay kit for implementing the method according to one of claims 14 and 15, comprising at least a protein preparation according to one of claims 1 to 5, and buffer solutions for implementing the method.

17. Kit according to claim 16 **characterized in that** it comprises a reagent for revealing the antibody-protein complex formed.


### LISTE DE SEQUENCES

(1) INFORMATIONS GENERALES:
   (i) DEPOSANT:
      (A) NOM: INSTITUT PASTEUR
      (B) RUE: 28, rue du Docteur Roux
      (C) VILLE: PARIS
      (E) PAYS: FRANCE
      (F) CODE POSTAL: 75724
      (G) TELEPHONE: 4 568 8000
      (H) TELECOPIE: 43 06 98 35
      (I) TELEX: 250609 F
   (ii) TITRE DE L' INVENTION: PROTEINES DE MYCOBACTERIES,MICROORGANISMES LES PRODUISANT ET LEURS UTILISATIONS VACCINALES ET POUR LA DETECTION DE LA TUBERCULOSE
   (iii) NOMBRE DE SEQUENCES: 3
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 2061 paires de bases
      (B) TYPE: nucléotide
      (C) NOMBRE DE BRINS: double
      (D) CONFIGURATION: linéaire
   (ii) TYPE DE MOLECULE: ADN (génomique)
   (iii) HYPOTHETIQUE: NON
   (iv) ANTI-SENS: NON
   (ix) CARACTERISTIQUE:
      (A) NOM/CLE: CDS
      (B) EMPLACEMENT:1082..2057
      (xi) DESCRIPTION DE LA SEQUENCE: SEQ ID NO: 1:
   GTGCTCGGGC CCAACGGTGC GGGCAAGTCC ACCGCCCTGC ATGTTATCGC GGGGCTGCTT      60
   CGCCCCCGAC GCGGGCTTGG TACGTTTGGG GGACCGGGTG TTGACCGACA CCGAGGCCGG     120
   GGTGAATGTG GCGACCCACG ACCGTCGAGT CGGGCTGCTG TTGCAAGACC CGTTGTTGTT     180
   TCCACACCTG AGCGTGGCCA AAAACGTGGC CTTCGGACCA CAATGCCGTC GCGGGATGTT     240
   TGGGTCCGGG CGCGCGCTAG GACAAGGGCG TCGGCACTGC GATGGCTGCG CGAGGTGAAC     300
   GCCGAGCAGT TCGCCGACCG TAAGCCTCGT CAGCTATCCG GGGGCCAAGC CCAGCGCGTC     360
   GCCATCGCGC GAGCGTTGGC GGCCGAACCG GATGTGTTGC TGCTCGACGA GCCGCTGACC     420
   GGACTCGATG TGGCCGCGGC CGCGGGTATC CGTTCGGTGT TGCGTAGTGT CGTCGCGAGG     480
   AGCGGTTGCG CGGTAGTCCT GACGACCCAT GACCTGCTGG ACGTGTTCAC GCTGGCCGAC     540
   CGGGTATTGG TGCTCGAGTC CGGCACGATC GCCGAGATCG GCCCGGTTGC CGATGTGCTT     600
   ACCGCACCTC GCAGTCGTTT CGGAGCCCGT ATCGCCGGAG TCAACCTGGT CAATGGGACC     660
   ATTGGTCCGG ACGGCTCGCT GCGCACCCAG TCCGGCGCCC ACTGGTACGG CACCCCGGTC     720
   CAGGATTTGC CTACTGGGCA TGAGGCAATC GCGGTGTTCC CGCCGACGGC GGTGGCGGTG     780
   TATCCGGAAC CGCCGCACGG AAGCCCGCGC AATATCGTCG GGCTGACGGT GGCGGAGGTG     840
   GATACCCGCG GACCCACGGT CCTGGTGCGC GGGCATGATC AGCCTGGTGG CGCGCCTGGC     900
   CTTGCCGCAT GCATCACCGT CGATGCCGCC ACCGAACTGC GTGTGGCGCC CGGATCGCGC     960
   GTGTGGTTCA GCGTCAAGGC GCAGGAAGTG GCCCTGCACC CGGCACCCCA CCAACACGCC    1020
   AGTTCATGAG CCGACCCGCG CCGTCCTTGC GTCGCGCCGT TAACACGGTA GGTTCTTCGC    1080
   C ATG CAT CAG GTG GAC CCC AAC TTG ACA CGT CGC AAG GGA CGA TTG        1126
     Met His Gln Val Asp Pro Asn Leu Thr Arg Arg Lys Gly Arg Leu
     1               5                   10                  15
   GCG GCA CTG GCT ATC GCG GCG ATG GCC AGC GCC AGC CTG GTG ACC GTT      1174
   Ala Ala Leu Ala Ile Ala Ala Met Ala Ser Ala Ser Leu Val Thr Val
                   20                  25                  30
   GCG GTG CCC GCG ACC GCC AAC GCC GAT CCG GAG CCA GCG CCC CCG GTA      1222
   Ala Val Pro Ala Thr Ala Asn Ala Asp Pro Glu Pro Ala Pro Pro Val
               35                  40                  45
   CCC ACA ACG GCC GCC TCG CCG CCG TCG ACC GCT GCA GCG CCA CCC GCA      1270
   Pro Thr Thr Ala Ala Ser Pro Pro Ser Thr Ala Ala Ala Pro Pro Ala
           50                  55                  60
   CCG GCG ACA CCT GTT GCC CCC CCA CCA CCG GCC GCC GCC AAC ACG CCG 1318
   Pro Ala Thr Pro Val Ala Pro Pro Pro Pro Ala Ala Ala Asn Thr Pro
   65 70 75
   AAT GCC CAG CCG GGC GAT CCC AAC GCA GCA CCT CCG CCG GCC GAC CCG 1366
   Asn Ala Gln Pro Gly Asp Pro Asn Ala Ala Pro Pro Pro Ala Asp Pro
   80 85 90 95
   AAC GCA CCG CCG CCA CCT GTC ATT GCC CCA AAC GCA CCC CAA CCT GTC 1414
   Asn Ala Pro Pro Pro Pro Val Ile Ala Pro Asn Ala Pro Gln Pro Val
   100 105 110
   CGG ATC GAC AAC CCG GTT GGA GGA TTC AGC TTC GCG CTG CCT GCT GGC 1462
   Arg Ile Asp Asn Pro Val Gly Gly Phe Ser Phe Ala Leu Pro Ala Gly
   115 120 125
   TGG GTG GAG TCT GAC GCC GCC CAC TTC GAC TAC GGT TCA GCA CTC CTC 1510
   Trp Val Glu Ser Asp Ala Ala His Phe Asp Tyr Gly Ser Ala Leu Leu
   130 135 140
   AGC AAA ACC ACC GGG GAC CCG CCA TTT CCC GGA CAG CCG CCG CCG GTG 1558
   Ser Lys Thr Thr Gly Asp Pro Pro Phe Pro Gly Gln Pro Pro Pro Val
   145 150 155
   GCC AAT GAC ACC CGT ATC GTG CTC GGC CGG CTA GAC CAA AAG CTT TAC 1606
   Ala Asn Asp Thr Arg Ile Val Leu Gly Arg Leu Asp Gln Lys Leu Tyr
   160 165 170 175
   GCC AGC GCC GAA GCC ACC GAC TCC AAG GCC GCG GCC CGG TTG GGC TCG 1654
   Ala Ser Ala Glu Ala Thr Asp Ser Lys Ala Ala Ala Arg Leu Gly Ser
   180 185 190
   GAC ATG GGT GAG TTC TAT ATG CCC TAC CCG GGC ACC CGG ATC AAC CAG 1702
   Asp Met Gly Glu Phe Tyr Met Pro Tyr Pro Gly Thr Arg Ile Asn Gln
   195 200 205
   GAA ACC GTC TCG CTC GAC GCC AAC GGG GTG TCT GGA AGC GCG TCG TAT 1750
   Glu Thr Val Ser Leu Asp Ala Asn Gly Val Ser Gly Ser Ala Ser Tyr
   210 215 220
   TAC GAA GTC AAG TTC AGC GAT CCG AGT AAG CCG AAC GGC CAG ATC TGG 1798
   Tyr Glu Val Lys Phe Ser Asp Pro Ser Lys Pro Asn Gly Gln Ile Trp
   225 230 235
   ACG GGC GTA ATC GGC TCG CCC GCG GCG AAC GCA CCG GAC GCC GGG CCC 1846
   Thr Gly Val Ile Gly Ser Pro Ala Ala Asn Ala Pro Asp Ala Gly Pro
   240 245 250 255
   CCT CAG CGC TGG TTT GTG GTA TGG CTC GGG ACC GCC AAC AAC CCG GTG 1894
   Pro Gln Arg Trp Phe Val Val Trp Leu Gly Thr Ala Asn Asn Pro Val
   260 265 270
   GAC AAG GGC GCG GCC AAG GCG CTG GCC GAA TCG ATC CGG CCT TTG GTC 1942
   Asp Lys Gly Ala Ala Lys Ala Leu Ala Glu Ser Ile Arg Pro Leu Val
   275 280 285
   GCC CCG CCG CCG GCG CCG GCA CCG GCT CCT GCA GAG CCC GCT CCG GCG 1990
   Ala Pro Pro Pro Ala Pro Ala Pro Ala Pro Ala Glu Pro Ala Pro Ala
   290 295 300
   CCG GCG CCG GCC GGG GAA GTC GCT CCT ACC CCG ACG ACA CCG ACA CCG 2038
   Pro Ala Pro Ala Gly Glu Val Ala Pro Thr Pro Thr Thr Pro Thr Pro
   305 310 315
   CAG CGG ACC TTA CCG GCC T GACC 2061
   Gln Arg Thr Leu Pro Ala
   320 325
(2) INFORMATIONS POUR LA SEQ ID NO: 2:
   (i) CARACTERISTIQUES DE LA SEQUENCE:
      (A) LONGUEUR: 325 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met His Gln Val Asp Pro Asn Leu Thr Arg Arg Lys Gly Arg Leu Ala
   1 5 10 15
   Ala Leu Ala Ile Ala Ala Met Ala Ser Ala Ser Leu Val Thr Val Ala
   20 25 30
   Val Pro Ala Thr Ala Asn Ala Asp Pro Glu Pro Ala Pro Pro Val Pro
   35 40 45
   Thr Thr Ala Ala Ser Pro Pro Ser Thr Ala Ala Ala Pro Pro Ala Pro
   50 55 60
   Ala Thr Pro Val Ala Pro Pro Pro Pro Ala Ala Ala Asn Thr Pro Asn
   65 70 75 80
   Ala Gln Pro Gly Asp Pro Asn Ala Ala Pro Pro Pro Ala Asp Pro Asn
   85 90 95
   Ala Pro Pro Pro Pro Val Ile Ala Pro Asn Ala Pro Gln Pro Val Arg
   100 105 110
   Ile Asp Asn Pro Val Gly Gly Phe Ser Phe Ala Leu Pro Ala Gly Trp
   115 120 125
   Val Glu Ser Asp Ala Ala His Phe Asp Tyr Gly Ser Ala Leu Leu Ser
   130 135 140
   Lys Thr Thr Gly Asp Pro Pro Phe Pro Gly Gln Pro Pro Pro Val Ala
   145 150 155 160
   Asn Asp Thr Arg Ile Val Leu Gly Arg Leu Asp Gln Lys Leu Tyr Ala
   165 170 175
   Ser Ala Glu Ala Thr Asp Ser Lys Ala Ala Ala Arg Leu Gly Ser Asp
   180 185 190
   Met Gly Glu Phe Tyr Met Pro Tyr Pro Gly Thr Arg Ile Asn Gln Glu
   195 200 205
   Thr Val Ser Leu Asp Ala Asn Gly Val Ser Gly Ser Ala Ser Tyr Tyr
   210 215 220
   Glu Val Lys Phe Ser Asp Pro Ser Lys Pro Asn Gly Gln Ile Trp Thr
   225 230 235 240
   Gly Val Ile Gly Ser Pro Ala Ala Asn Ala Pro Asp Ala Gly Pro Pro
   245 250 255
   Gln Arg Trp Phe Val Val Trp Leu Gly Thr Ala Asn Asn Pro Val Asp
   260 265 270
   Lys Gly Ala Ala Lys Ala Leu Ala Glu Ser Ile Arg Pro Leu Val Ala
   275 280 285
   Pro Pro Pro Ala Pro Ala Pro Ala Pro Ala Glu Pro Ala Pro Ala Pro
   290 295 300
   Ala Pro Ala Gly Glu Val Ala Pro Thr Pro Thr Thr Pro Thr Pro Gln
   305 310 315 320
   Arg Thr Leu Pro Ala
   325
(2) INFORMATIONS POUR LA SEQ ID NO: 3:
   (i) CARACTERISTIQUES DE LA SEQUENCE:
      (A) LONGUEUR: 286 acides aminés
      (B) TYPE: acide aminé
      (C) NOMBRE DE BRINS:
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 3:
   Asp Pro Glu Pro Ala Pro Pro Val Pro Thr Thr Ala Ala Ser Pro Pro
   1 5 10 15
   Ser Thr Ala Ala Ala Pro Pro Ala Pro Ala Thr Pro Val Ala Pro Pro
   20 25 30
   Pro Pro Ala Ala Ala Asn Thr Pro Asn Ala Gln Pro Gly Asp Pro Asn
           35                  40                  45
   Ala Ala Pro Pro Pro Ala Asp Pro Asn Ala Pro Pro Pro Pro Val Ile
       50                  55                  60
   Ala Pro Asn Ala Pro Gln Pro Val Arg Ile Asp Asn Pro Val Gly Gly
   65                  70                  75                  80
   Phe Ser Phe Ala Leu Pro Ala Gly Trp Val Glu Ser Asp Ala Ala His
                   85                  90                  95
   Phe Asp Tyr Gly Ser Ala Leu Leu Ser Lys Thr Thr Gly Asp Pro Pro
               100                 105                 110
   Phe Pro Gly Gln Pro Pro Pro Val Ala Asn Asp Thr Arg Ile Val Leu
           115                 120                 125
   Gly Arg Leu Asp Gln Lys Leu Tyr Ala Ser Ala Glu Ala Thr Asp Ser
       130                 135                 140
   Lys Ala Ala Ala Arg Leu Gly Ser Asp Met Gly Glu Phe Tyr Met Pro
   145                 150                 155                 160
   Tyr Pro Gly Thr Arg Ile Asn Gln Glu Thr Val Ser Leu Asp Ala Asn
                   165                 170                 175
   Gly Val Ser Gly Ser Ala Ser Tyr Tyr Glu Val Lys Phe Ser Asp Pro
               180                 185                 190
   Ser Lys Pro Asn Gly Gln Ile Trp Thr Gly Val Ile Gly Ser Pro Ala
           195                 200                 205
   Ala Asn Ala Pro Asp Ala Gly Pro Pro Gln Arg Trp Phe Val Val Trp
       210                 215                 220
   Leu Gly Thr Ala Asn Asn Pro Val Asp Lys Gly Ala Ala Lys Ala Leu
   225                 230                 235                 240
   Ala Glu Ser Ile Arg Pro Leu Val Ala Pro Pro Pro Ala Pro Ala Pro
                   245                 250                 255
   Ala Pro Ala Glu Pro Ala Pro Ala Pro Ala Pro Ala Gly Glu Val Ala
               260                 265                 270
   Pro Thr Pro Thr Thr Pro Thr Pro Gln Arg Thr Leu Pro Ala
           275                 280                 285